(19) Europäisches Patentamt
European Patent Office
Office européen des brevets

(11) EP 2 687 537 B1

(12) # EUROPEAN PATENT SPECIFICATION

(45) Date of publication and mention of the grant of the patent:
**15.11.2017 Bulletin 2017/46**

(21) Application number: **12757419.2**

(22) Date of filing: **15.03.2012**

(51) Int Cl.:
***C07K 7/08*** (2006.01) ***C12N 15/11*** (2006.01)
***C12N 15/63*** (2006.01) ***A61K 38/10*** (2006.01)
***A61P 1/16*** (2006.01) ***A61P 35/00*** (2006.01)

(86) International application number:
**PCT/CN2012/072380**

(87) International publication number:
**WO 2012/122941 (20.09.2012 Gazette 2012/38)**

(54) **POLYPEPTIDE DRUG AGAINST HEPATITIS B VIRUS X PROTEIN**

POLYPEPTID-ARZNEIMITTEL GEGEN DAS HEPATITIS-B-VIRUS-X-PROTEIN

MÉDICAMENT CONTRE LA PROTÉINE X DU VIRUS DE L'HÉPATITE B UTLISANT UN POLYPEPTIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2011 CN 201110061840**

(43) Date of publication of application:
**22.01.2014 Bulletin 2014/04**

(73) Proprietor: **Tianjin Toptech Bio-science&technology Co., Ltd.**
**Tianjin 300457 (CN)**

(72) Inventors:
- **ZHANG, Xiaodong**
  **Tianjin 300071 (CN)**
- **YE, Lihong**
  **Tianjin 300071 (CN)**

(74) Representative: **Engelhard, Markus**
**Boehmert & Boehmert**
**Anwaltspartnerschaft mbB**
**Pettenkoferstrasse 22**
**80336 München (DE)**

(56) References cited:
**WO-A1-2007/110098 WO-A2-2010/017515**

- **DATABASE GENBANK [Online] 13 December 2010 MELEGARI, M. ET AL.: 'Cloning and Characterization of a Novel Hepatitis B Virus x Binding Protein That Inhibits Viral Replication', XP003030196 Database accession no. NP_006393.2 & JOURNAL OF VIROLOGY vol. 72, no. 3, March 1998, pages 1737 - 1743**
- **WANG, F. ET AL.: 'Involvement of hepatitis B X-interacting protein (HBXIP) in proliferation regulation of cells' ACTAPHARMACOL SIN vol. 28, no. 3, March 2007, pages 431 - 438, XP055124197**
- **WANG, FENGZE ET AL.: 'Promotion of Transcriptional Activity of NF-KB Mediated by HBXIP in Hepatoma Cells' PROGRESS IN BIOCHEMISTRY AND BIOPHYSICS vol. 34, no. 11, 15 November 2007, pages 1197 - 1201, XP055124198**
- **DATABASE GENESEQ [Online] 13 May 2010 Database accession no. AXW79225**

EP 2 687 537 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Related Applications

This application claims the benefit of Chinese Patent Application No. 201110061840.5, filed on March 15, 2011, and entitled "Polypeptide Drug against Hepatitis B Virus X Protein," which is incorporated herein by reference in its entirety.

### Technical Field

The present invention relates to the technology of polypeptide medicine, and especially relates to the polypeptides and polynucleotides encoding these polypeptides, and methods of use.

### Background

Liver cancer (Hepatocellular Carcinoma, HCC) is one of the malignant tumors that can lead to death. According to the statistics, in China, the mortality rate of liver cancer is only less than the mortality rate of gastric cancer. Each year, there are about 300,000 people who are diagnosed as having liver cancer, and about 110,000 people die from this disease.

Hepatitis B Virus (HBV) may lead to hepatitis, hepatic cirrhosis and primary liver cancer. The HBV is a DNA virus of about 3.2Kb, and it contains overlapping open reading frames (ORFs) that are responsible for transcription and expression of the hepatitis B virus surface antigen (HBsAg), the hepatitis B virus core antigen (HBcAg), the hepatitis B virus polymerase, and the hepatitis B virus X antigen (HBxAg) (or so-called hepatitis B virus X protein, HBx). Among them, HBx is essential for the HBV DNA to replicate. Therefore, to inhibit the function of HBx may lead to suppression of the HBV infection, as well as the subsequent hepatitis and cirrhosis.

In addition, as a trans-activator factor, HBx can promote the growth and proliferation of liver cancer, which is known as the oncoprotein. Studies at the molecular level, cellular level and *in vivo* level have shown that HBx has strong effects in promoting liver cancer cells's proliferation and migration. Transgenic mouse experiments also showed that HBx can cause liver cancers. The results of numerous studies further indicated that the continuous HBV infection could lead to chronic liver diseases, including chronic hepatitis, repeated chronic hepatitis, which subsequently causes hyperplasia of fibrous connective tissue in liver tissues and cirrhosis, and liver cancer based on cirrhosis. Therefore, it has been well established that HBx plays a critical role in the development and progress of chronic liver diseases (including hepatitis, cirrhosis and liver cancer). Consequently, HBx becomes an important target for preventing and treating liver diseases.

Currently, the treatment of liver cancer is mainly through surgery, supplemented with interventional therapies, whereas chemotherapy is usually not very effective. Clinical data show that the rate of finding HBsAg and HBxAg expressed in liver cancer tissues is more than 80% or even 90%. Since HBx is an important pathogenic factor in the occurrence and development of liver cancer, identifying and developing its specific inhibitors would have great theoretical and clinical significance. However, due to lack of three-dimensional structure of HBx, it is rather difficult to design its chemical inhibitors through HBx's three-dimensional conformation information.

Fragmental polypeptides can be used as medicine, and they have already been widely used in clinical practice. For example, thymopeptide is a thymopentin extracted from calf thymus, with the function of promoting lymphocyte transformation, enhancing phagocytic activity of macrophages, and can be used to treat a variety of immunodeficiency diseases. The characteristics of the polypeptide drugs include having clear pharmacokinetics effects, safe, and easy to be manufactured. However, proper polypeptide drugs are difficult to be discovered, and most of the polypeptide fragments have very short half-life *in vivo*, which affects the pharmacokinetics effect of the drug directly.

### Summary of Invention

The present invention relates to a group of polypeptides that can inhibit the functional activity of the hepatitis B virus X protein (hepatitis B virus X protein, HBx), it can inhibit the activity of the HBx at the molecular level, the cellular level and *in vivo*, and thus can inhibit hepatitis caused by the hepatitis B virus infection, cirrhosis caused by repeated hepatitis infection, and liver cancers developed based on cirrhosis. The polypeptides and the mimetics thereof, including their functional fragments and variants, as well as the genes encoding these polypeptides and the peptidomimetics and their functional fragments and variants, can be widely used for the prevention and treatment of the liver diseases resulted from hepatitis B infection, including hepatitis, cirrhosis and liver cancer.

In one aspect, the present invention provides isolated polypeptides or peptidomimetics as defined by any of claims 1-4. The chronic liver diseases resulted from hepatitis B virus infection comprise the hepatitis, hepatic cirrhosis caused by repeated hepatitis infection, and liver cancers developed from cirrhosis.

In some embodiments of present invention, said polypeptides or peptidomimetics, or the functional fragments and functional variants thereof, comprise amino acid sequence that have at least 70% identity to the amino acid sequence shown in SEQ ID NO: I, or at least 80% identity, or at least 90% identity, or even higher. Preferably, said polypeptides or peptidomimetics comprise any one of the amino acid sequence shown in SEQ ID NOs: 1-6.

In another aspect, the present invention provides isolated polynucleotides as defined by any of claims 5-6. Preferably, said polynucleotides are the polynucleotides that encode any one of the amino acid sequences as shown in SEQ ID NOs: 1-6; alternatively, said polynucleotides are the polynucleotides that are complementary to the polynucleotides encoding any one of the amino acid sequences as shown in SEQ ID NOs: 1-6, or can hybridize with them under stringent hybridization condition.

In another aspect, the present invention also provides a recombinant expression vector as defined by claim 7. Preferably, said polynucleotide is the polynucleotide encoding any one of the amino acid sequences as shown in SEQ ID NOs: 1-6; or a polynucleotide that is either complementary thereto or capable of hybridizing therewith under stringent hybridization condition. The present application also provides A host cells as defined by claim 8.
The present invention also provides the polypeptide for use according to claim 9, the polynucleotide for use according to claim 10, the recombination expression vector for use according to claim 11 and a therapeutic vaccine for use according to claim 12.

In addition, the present invention also provides usage of the polypeptide or peptidomimetics, the polynucleotides, and the recombinant expression vectors in the manufacture of medicines for treating and preventing chronic liver diseases resulted from the hepatitis B virus infection. In a specific embodiment, said medicine is the hepatitis B virus vaccine. Said medicines comprise pharmaceutical compositions, which may contain optional pharmaceutical carrier.

The present invention also provides a pharmaceutical composition as defined by any of claims 13-15.

In the present invention, said polypeptide (including its functional fragment or variant thereof) and its peptidomimetic, as an effective inhibitory factor of HBx, is capable of inhibiting the biological activities of HBx, and can inhibit the hepatitis caused by hepatitis B virus infection, as well as the cirrhosis and liver cancers developed therefrom. It is worth of emphasizing that above-described polypeptides provided by the present invention have significant pharmacokinetics effects, and therefore can be used as effective drugs for the treatment and prevention of liver diseases caused by the hepatitis B virus infection.

## Brief Description of Drawings

Figure 1. Analysis result of purified artificial synthesized polypeptide anti-HBxP1# by High Pressure Liquid Chromatography (HPLC).

Figure 2. Analysis of the effects of the plasmids containing the invention polypeptide genes on the expression of HBx protein in the hepatoma cells by reporter gene assay. Results show that plasmid p-Anti-HBxP1# inhibits the activity of NF-κB promoter in the HepG2-X cells, L-O2-X cells and HepG2.2.15 cells in a dose-dependent manner, and has no effect on the hepatoma cells HepG2 and L-O2 which do not express HBx protein. Similarly, the experiments further indicate that the plasmids containing genes encoding five functional variants of Anti-HBxP1# (0.15μg/well) also inhibit the activities of NF-κB promoter in the HepG2-X cells and L-O2-X cells. Student's t-test is employed for statistical analysis, * $P<0.05$, ** $P<0.01$.

Figure 3. Analysis of the effects of the invention polypeptides on the activity of NF-κB promoter by HBx protein at the cellular level. Results show that incubation with artificial synthesized polypeptide Anti-HBxP1# inhibits the activity of NF-κB promoter in the HepG2-X cells, L-O2-X cells and HepG2.2.15 cells in a dose-dependent manner, but has no effect on the hepatoma cells HepG2 and L-O2 which do not express HBx protein. Student's *t*-test is employed for statistical analysis, * $P<0.05$, ** $P<0.01$.

Figure 4. Analysis of the effects of the plasmids containing invention polypeptide genes on the expression of HBx protein in the hepatoma cells by MTT assay. Results show that p-anti-HBxP1# inhibits the activity of NF-κB promoter in the HepG2-X cells, L-O2-X cells and HepG2.2.15 cells in a dose-dependent manner, but has no effect on the hepatoma cells HepG2 and hepatoma cells L-O2 which do not express HBx. Similarly, the genes containing 5 variants of Anti-HBxP1# (0.15μg/well) inhibit the activity of NF-κB promoter in the HepG2-X cells and L-O2-X cells. Student's *t*-test is employed for statistical analysis, * $P<0.05$, ** $P<0.01$.

Figure 5. Analysis of the effects of the invention polypeptides on the growth of the hepatoma cells by MTT assay. Results show that artificial synthesized polypeptide Anti-HBxP1# inhibits the activity of NF-κB promoter in the HepG2-X cells, L-O2-X cells and HepG2.2.15 cells in a dose-dependent manner, but has no effect on the hepatoma cells HepG2 and hepatoma cells L-O2 which do not express HBx. Student's t-test is employed for statistical analysis, * P<0.05, ** P<0.01.

Figure 6. The effect of synthesized invention polypeptide anti-HBxP1# on the growth of HepG2-X cells. The results of inoculation experiment in nude mice shows that Anti-HBxP1# inhibits the growth and proliferation in the HepG2-X cells. Student's t-test is employed for statistical analysis, ** P<0.01.

Figure 7. The effect of synthesized polypeptide anti-HBxP1# on the growth of HepG2.2.15 cells. The results of inoculation experiment in nude mice shows that Anti-HBxP1# inhibits the growth and proliferation in the HepG2.2.15 cells. Student's t-test is employed for statistical analysis, ** P<0.01.

Figure 8. Schematic diagram of the construction of the eukaryotic expression vector containing the gene encoding the invention polypeptide.

**Detailed Description**

In the present invention, including the description and claims, unless otherwise specified, the following terms are used with the following meanings:

"Isolated" refers to separating a substance from its original environment (e.g., its natural environment if it is naturally generated). For example, a naturally generated polynucleotide or polypeptide existing in a live animal means it has not been separated, whereas the same polynucleotide or polypeptide separated partially or completely from the natural systems with which it usually coexist means it is separated. Such a polynucleotide or polypeptide may exist as a part of a vector, or a part of a composition; since the vector and the composition are not a component of its natural environment, they are still separated.

The term "purified" as used herein means an increased status in purity , wherein "purity" is a relative term, and should not be narrowly construed as absolute purity. For example, the purity can be at least above about 50% , or greater than 60% , or than 70%, or than 80% , or than 90% , or even reach to 100%.

As used in the present invention, the isolated substance is separated from its original environment. The polynucleotides and polypeptides existed within the living cells are not isolated; however, the same polynucleotides and polypeptides that are separated from the substances that they usually coexist in the natural state should be regarded as be separated, while the purity is improved, and thus is purified.

The term "nucleic acid sequence", "nucleotide sequence" or "base pair sequence" used herein refer to nucleotide, oligonucleotide, polynucleotide and fragments or portions thereof. They generally refers to DNA (e.g., cDNA or genomic DNA) or RNA (e.g., mRNA), which can be single-stranded or double-stranded. Single-stranded DNA or RNA can be coding strand (sense strand) or noncoding strand (antisense strand). Additionally, a polynucleotide referred in the present invention can also at its 5' terminal or 3' terminal be fused with tag (tag sequence or marker sequence). Synthesized or obtained (e.g., isolated and/or purified) from natural sources, they can contain natural, non-natural or altered nucleotides, and can contain natural, non-natural or altered internucleotide bond, such as a phosphoroamidate bond or a phosphorothioate bond , instead of the phosphodiester bond found between the nucleotides of an unmodified oligonucleotide.

The so-called "amino acid sequence" or "polypeptide" refers to a peptide, oligopeptide, polypeptide or protein and their partial fragment , which consists of amino acids that are connected with each other by peptide bonds. When amino acid sequence in the present invention is related to the amino acid sequence of a naturally occurred protein molecule, "polypeptide" or "protein" is not meant to limit the amino acid sequence for the entire natural amino acid sequence of said protein molecules. The amino acid sequence of the present invention may contain additional peptides. Labeled peptide epitope can be additional peptides, such as multiple histidine tag (His-tag), or myc, flag, etc.

"Deletion" refers to the deletion of one or a plurality of amino acids or nucleotides from the amino acid sequence or nucleotide sequence, respectively.

"Insert" or "add" refers to the increasing of one or a plurality of amino acids or nucleotides caused by the change of amino acid sequence or nucleotide sequence, respectively, compared with the molecules of the natural presence or before the change.

"Substitution" refers to one or a plurality of amino acids or nucleotides are replaced by different amino acids or nucleotides.

"Deletion, substitution or addition of one or a plurality of amino acids or nucleotides" refers to the use of known methods of mutating nucleic acid or polypeptide such as directed mutagenesis method to delete, substitute or add one or a plurality of the number of amino acid or nucleoside acid, or a naturally occurring nucleic acid or polypeptide of the mutation being separated and purified. Mutation of amino acids can contain one or a plurality of amino acid residues

with the conformation of D-amino acids, rare amino acids, naturally occurred or even artificially modified amino acids, and these amino acids may or may not be encoded by the genetic codon. Similarly, the induction of nucleic acid mutation can include the naturally occurring nucleotide, and may also include a modified nucleotide.

The "functional fragment" of a polypeptide as used herein refers to any part or portion of the polypeptide of the invention, which retains the substantially similar or identical biological activity of the polypeptide of which it is a part (the parent polypeptide).

The "functional variant" of a polypeptide as used herein refers to amino acid sequences having substantial similar or identical biological activity of a polypeptide, including, for example, 1) the original amino acid sequence with one or more amino acid residues deletion and/or one or more amino acid residues addition; or 2) one or more of the amino acid residues in the original amino acid sequence substituted by one or more conserved or non-conserved amino acid residues; or 3) a group in one or more amino acid residues of the amino acid sequence substituted by other group; or 4) the original amino acid sequence fused with another molecule or compound (such as sugar, lipids, polyethylene glycol, etc.); or 5) original amino acid sequence fused with additional polypeptide sequences (e.g., leader sequence or a secretion signal sequence or polypeptide sequence used for purifying purpose); or 6) the retroinverso analogue of the original amino acid sequence; or 7) mixed of above. In the present invention, the functional variant of an amino acid sequence may contain one or more D-type amino acids, rare amino acids that are naturally occurred, or artificially modified amino acids, these amino acids may or may not be encoded by the genetic code.

The term "retroinverso analogue" refers to a polypeptide comprising a revered amino acid sequence of a parent polypeptide, such that the amino acid sequence of the retroinverso analogue (when read from the N-terminus to the C-terminus) is the same as the amino acid sequence of the parent polypeptide when read from the C-terminus to the N-terminus. Furthermore, with respect to a retroinverso analogue, each of the amino acid is the D isomer of the amino acid, as opposed to the L isomer. For example, the retroinverso analogue of the tripeptide Val-Ala-Gly has an amino acid sequence Gly-Ala-Val, and each of the amino acid is the D isomer.

In the present invention, the term of "peptidomimetics" as used herein refers to a compound which has essentially the same general structure of a corresponding polypeptide with modifications that increase its stability or biological function. A peptidomimetics includes, for example, those compounds comprising the same amino acid sequence of a corresponding polypeptide with an altered backbone between two or more of the amino acids. The peptidomimetics can comprise synthetic or non-naturally occurred amino acids in place of naturally-occurred amino acids.

In the present invention, "degenerate variant" of the nucleotide sequence is such a polynucleotide sequence that is different from the parent nucleotide sequence, but encodes the same protein or polypeptide as the parent nucleotide sequence does.

"Nucleic Acid Hybridization" is known in the art (see, e.g., Sambrook et al, Molecular Cloning: A Laboratory Manual, 3rd Ed., Cold Spring Harbor Laboratory, 2001). In general, the higher the temperature is, the lower the salt concentration is, the more rigor the hybridization condition becomes (more difficult to hybridize), thereby obtaining more similar polynucleotides. The proper hybridization temperature varies depending on the length of the nucleotide sequence or its base-pair sequence. Further, the present invention also relates to hybridization under "stringent condition." The term "stringent conditions" in the present invention refers to hybridization and elution in a condition of low ionic concentration and high temperature. For example, incubation overnight at 42°C, (50% formamide, 5 x SSC (150 mM NaCl, 15 mM citric acid tri-sodium), 50 mM sodium phosphate (pH 7.6), 5 X Denhardt's solution, 10% sulfuric acid dextran, and 20 $\mu$g/ml of denatured cut salmon sperm DNA), and then elution with 0.1 X SSC at 65°C.

"Homology" refers to the degree of complementation, which may be partially homologous, or may be completely homologous. "Partial homologous" refers to a partially complementary sequence, which can partially inhibit hybridization between the target nucleotide with a completely complementary sequence. This inhibition may be detected by hybridization under reduced rigor condition (southern blot or northern blot, etc.). Substantially homologous sequence or hybridization probe can compete and inhibit the binding of the completely complementary sequence to the target sequence under reduced rigor condition. Of course, reduced rigor condition does not allow non-specific binding. The two sequences combined with each other still require a specific interaction.

The percentage of "homology" or "identity" of amino acid sequence or nucleotide sequence refers to a percentage of sequence identity or similarity in the comparison with two or more amino acid sequences or nucleotide sequences. There are many methods to determine the percentage of sequence identity for the skilled artisan, such as the MEGALIGN program (Lasergene Software Packages, DNASTA Inc., Madison, WI). MEGALIGN program can compare two or more sequences based on the different types of method such as the Cluster Method (see Higgins & Sharp, Gene 73:237-244 (1988))., Each set of sequence is aligned by clusters by checking the distance between the pairs, and then the cluster is assigned by pairs or groups. The percentage of similarity of two amino acid sequences, sequence A and sequence B, can be calculated by the following formula:

[(the number of residues matching between Sequence A and Sequence B) / (the number of residues of sequence A - residues of the sequence A in the intervals - residues of sequence B in the intervals)] X 100%

Similarly, it can be calculated by Cluster method or methods known in the art, such as the Jotun Hein method (see Hein J., the Methods in Emzumology 183:625-645, 1990) to determine the percentage of the similarity between two nucleic acid sequence.

The term "recombinant expression vector" means a genetically-modified recombinant oligonucleotide or polynucleotide, which comprises nucleotide sequence encoding mRNA, protein, polypeptide, and peptide when the recombinant vector is contacted with the host cell under conditions sufficient to have the mRNA, protein, polypeptide or peptide expressed within the cell. The invention recombinant expression vector can comprise any type of nucleotides, including, but not limited to DNA and RNA, which can be single-stranded or double-stranded, synthesized or obtained in part from natural sources , and which can contain natural, non-natural or altered nucleotides. The linkage between nucleotide can be naturally-occurring, and can also be non-naturally-occurring or modified.

In the present invention, "the chronic liver diseases resulted from hepatitis B virus infection" refers to liver diseases caused by hepatitis B virus infection, including chronic hepatitis, cirrhosis after hyperplasia of fiberous connective tissue in liver tissue caused by repeated hepatitis, and most of the liver cancers based on cirrhosis. The occurrence of liver cancer is a result of continuous infection with hepatitis B virus, and said liver cancer can be called as post hepatitis B liver cancer.

In the present invention, "treatment" and "prevention" and words derived from them, do not mean 100% or completely treatment or prevention, but can be identified as the treatment or prevention extent approved by those skilled. In the present invention, "prevention" could be understood as delay the onset of the disease, or its symptoms or disorders.

Polypeptide

The present invention relates to isolated or purified polypeptides. The polypeptides comprise, basically consist of, or consist of the amino acid sequence as follows: Gly-Ser-Ala-Val-Met-Phe-Ser-Ser-Lys-Glu-Arg-Gly (SEQ ID NO: 1). The study has confirmed that these polypeptides can significantly inhibit the activity of HBx, and have significant inhibitory effects on the occurrence and development of chronic liver diseases resulted from the hepatitis B virus infection. Of particular importance is these peptides greatly inhibit the growth and proliferation of hepatoma cells infected with hepatitis B virus.

The inhibition is reflected at the molecular level, the cellular level and the *in vivo* level. For example, said polypeptides at the molecular level can inhibit the HBx-activated nuclear factor κB (NF-κB) promoter activity, which suggests that said polypeptides effectively inhibit HBx, and the inhibitory effects thereof are dose-dependent; said polypeptides at the cellular level also effectively inhibit the growth and proliferation of hepatoma cells that contain HBx gene and the inhibitory effects thereof are also dose-dependent; nude mice inoculation experiments further show that the polypeptides can effectively inhibit the growth of the hepatoma cells that express HBx. However, the polypeptides show no clear effect on the NF-κB promoter activity in hepatoma cells that do not have HBx expression, and have no effects on the growth and proliferation of hepatoma cells that do not express HBx.

Herein described are also various functional fragments of the polypeptide. Said functional fragments can be any fragments of the continuous amino acid sequence of the polypeptide of the present invention on condition that the functional fragments can retain the parent polypeptide's biological activity by a similar extent, by the same extent, or by a higher extent compared with the parental polypeptide, e.g., inhibiting HBx activity. With reference to the parent polypeptide, the functional fragments can have, e.g., approximately 10%, 20%, 30%, 40%, 50%, 60%, 70%, 80%, 90%, 95%, 100%, 105%, 110%, 120%, 150%, 200% or even greater activity of that provided by the parent polypeptide. Said functional fragments can also contain additional amino acids at the amino or carboxyl terminus of the continuous amino acid sequence of the fragment thereof, or at both terminuses, for example, different amino acid sequence compared with the parent polypeptide. It is desirable that said additional amino acids do not hinder the biological function of said functional fragment, e.g., inhibition of the HBx activity, and capable of effectively inhibit cancer cells, preferably liver cancer cells, especially the growth and proliferation of liver cancer cell resulted from hepatitis B virus infection. More preferably, said additional amino acids can lead to enhanced biological activity when compared with the biological activity of the parent polypeptide. It is preferred that the functional fragments of the polypeptide of the present invention include the amino acid sequence having at least 70% sequence identity to said parent polypeptide. In a specific embodiment, a functional

fragment includes adding amino acids to the parent polypeptide amino terminal (N terminal) and/or carboxyl-terminal (C terminal), e.g. one or two amino acids.

The functional variants of the polypeptide and functional fragments thereof retains substantial similar or identical biological activity with the parent polypeptide or the functional fragments thereof, e.g., inhibition of the HBx activity, and subsequently effectively inhibit liver cancer cells, especially the cell growth and proliferation in liver cancers resulted from infection of hepatitis B virus. With reference to parent polypeptide or the functional fragment thereof, the functional fragment can have, e.g., approximately 50%, 60%, 70%, 80%, 90%, 95% or 100% identity in the amino acid sequence. Preferably, the functional variants of the polypeptide and functional fragments thereof include the sequence having at least 70% sequence identity with that of the parent polypeptide or the functional fragment thereof. More preferably, the functional variant of the invention polypeptide and the functional fragments thereof differ from the parent polypeptide and the functional fragment thereof by only 1-3 amino acids. In some particularly preferred embodiments, the functional variant of the polypeptide and functional fragments thereof differ from the parent polypeptide and the functional fragment thereof by only one amino acid.

The functional variant can, for example, comprise the amino acid sequence of the parent polypeptide or parent functional fragment with at least one conservative amino acid substitution. Specially, the functional variant can comprise the amino acid sequence of the parent polypeptide or parent functional fragment with two, three, four, five, or more conservative amino acid substitutions. Alternatively or additionally, the functional variants can comprise the amino acid sequence of the parent polypeptide or parent functional fragment with at least one non-conservative amino acid substitution. Specially, the functional variants can comprise the amino acid sequence of the parent polypeptide or the functional fragment thereof with two, three, four, five, or more non-conservative amino acid substitutions. In this case, it is preferable for the non-conservative amino acid substitutions to not interfere with or inhibit the biological activities of the functional variants. Preferably, the non-conservative amino acid substitution enhances the biological activity of the functional variant, such that the biological activity of the functional variant is increased as compared to the parent polypeptide or functional fragment thereof.

The polypeptides in this invention and their functional variants preferably comprise one or more conservative amino acid substitutions. Conservation amino acids substitutions are known in the art, and include amino acid substitutions in which one amino acid having certain physical and/or chemical properties is exchanged for another amino acid that has the same chemical or physical properties. Skilled in the art understand that conservative amino acid substitutions can not cause significant changes in the structure or function of the protein. For instance, the conservative amino acid substitution can be an acidic amino acid substituted for another acidic amino acid (e.g., Asp or Glu), an amino acid with a nonpolar side chain substituted for another amino acid with a nonpolar side chain (e.g., Ala, Gly, Val, Ile, Leu, Met, Phe, Pro, Trp, Val, etc.), a basic amino acid substituted for another basic amino acid (Lys, Arg, etc.), an amino acid with a polar side chain substituted for another amino acid with a polar side chain (Asn, Cys, Gln, Ser, Thr, Tyr, etc.), an aromatic amino acid (Trp, Phe, Tyr, etc.) for another aromatic amino acid, etc.

Alternatively, the functional variants may comprise a retroinverso analogue of any of the invention polypeptides or functional fragments thereof. The term "retroinverso analogue" refers to a polypeptide comprising a revered amino acid sequence of a parent polypeptide, such that the amino acid sequence of the retroinverso analogue (when read from the N-terminus to the C-terminus) is the same as the amino acid sequence of the parent polypeptide when read from the C-terminus to the N-terminus. Furthermore, with respect to the retroinverso analogue, each of the amino acid is the D isomer of the amino acid, as opposed to the L isomer. For example, the retroinverso analogue of the tri-peptide Val-Ala-Gly has an amino acid sequence Gly-Ala-Val, in which each amino acid is the D isomer. With respect to this invention, the functional variants preferably comprise a retroinverso analogue of SEQ ID NO: 1.

The polypeptides (including the functional fragments thereof) and their functional variants can be of any length, i.e., can comprise any number of amino acids , provided that the polypeptide (or the functional fragments thereof) and their functional variants retain the essential biological activities of the parent polypeptide, e.g., the ability to inhibit the activity of HBx, and effectively inhibit cancer cells, preferably liver cancer cells, especially the liver cancer cells the resulted from the hepatitis B virus infection. For example, the invention polypeptide or the peptidomimetic can be 50 to 5000 amino acids long, such as 5 , 6 , 7 , 8 , 9 , 10 , 11 , 12 , 13 , 14 , 15 ,16 , 20 , 25 , 30 , 40, 50 , 70, 75 , 100 , 125 , 150 , 175 , 200 , 300 , 400 , 500 , 600 , 700 , 800, 900, 1000 or more amino acids in length. Preferably, the polypeptides of the invention are 6 to 20 amino acids in length, and meet the requirements of the pharmacokinetics and half-life of polypeptide drug.

In one embodiment, the functional variant of the parent polypeptide and the functional fragments thereof have only one amino acid different from the parent polypeptide of SEQ ID NO: 1, and have very similar biological activity and function with the parent polypeptide of SEQ ID NO: 1, for example, inhibition of the activity of HBx, and effectively inhibiting cancer cells, preferably liver cancer cells, especially the hepatoma cells that express HBx protein. More specifically, a functional variant of the invention polypeptide and the functional fragments thereof include any of SEQ ID NOs: 2-6.

Also provided by the invention are peptidomimetics of any of the invention polypeptides described herein. In a

preferred embodiment, the peptidomimetics is a peptoid. The term "peptoid" as used herein refers to a peptidomimetics in which the side chains of each amino acid are appended to the nitrogen atom of the amino acid as opposed to the alpha carbon. For example, peptoids can be considered as N-substituted glycines which have repeating units of the general structure of $NRCH_2CO$ and which have the same or substantially the same amino acid sequence as the corresponding polypeptide. In another preferred embodiment, the peptidomimetics comprises an altered backbone in which the bond between each amino acid is methyalted. In this regard, the peptidomimetics can comprise a methylated peptide backbone of the following structure:

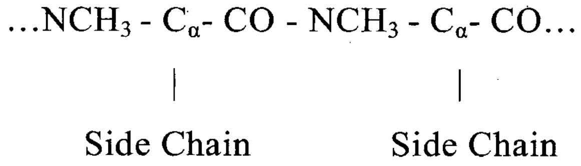

The polypeptide and peptidomimetics of the invention can comprise synthetic amino acid in place of one or more naturally-occurred amino acids. Such synthetic amino acids are known in the art, and include, for example, aminocyclohexane carboxylic acid, norleucine, α-amino n-decanoic acid, homoserine, S-acetylaminomethyl-cysteine, trans-3-hydroxyproline, trans-4-hydroxyproline, 4-aminophenylalanine, 4-benzoylphenylalanine, 4-nitropheylalanine, 4-chlorophenylalanine, 4-carboxyphenylalanine, β-phenylserine, β-hydroxyphenylalanine, phenylglycine, α-naphthylalanine, cyclohexylalanine, cyclohexylglycine, indoline-2-carboxylic acid, 1,2,3,4-tetrahydroisoquinoline-3-carboxylic acid, aminomalonic acid, aminomaloinc acid monoamide, N'-benzyl-N'-methyl-lysine, N',N'-dibenzyl-lysine, 6-hydroxylysine, ornithine, α-aminocyclopentane carboxylic acid, α-aminocyclohexane carboxylic acid, α-aminocycloheptane carboxylic acid, α-(2-amino-2-norbornane)-carboxylic acid, α,γ-diaminobutyric acid, α,β-diaminopropionic acid, homophenylalanine, and α-tert-butylglycine.

The invention polypeptides or the peptidomimetics thereof may also comprise a cell-penetrating peptide (CPP). Such a CPP facilitates the entry of the invention polypeptide or peptidomimetics across the cell membrane and into the cell. CPPs are known in the art (See, for example, Deshayes et al., Cell. Mol. Life Sci. 62:1839-1849 (2005); El-Andaloussi et al., Curr. Pharm. Design.11:3597-3611(2005)). The CPP described herein can be any of those known in the art.

The polypeptides and peptidomimetics of the invention can be lipidated (e.g., fatty acidated), glycosylated, amidated, carboxylated, phosphorylated, esterified, N-acylated, cyclized via, e.g., a disulfide bridge, or converted into an acid addition salt and/or optinally dimerized or polymerized, and/or conjugated.

For instance, the polypeptides and peptidomimetics can be lipidated derivatives. The lipid can be any lipid known in the art, such as, for example, a fatty acid, a farnesyl group (e.g., farnesyl diphosphate), a geranylgeranyl group (e.g., geranylgeranyl diphosphate), a phospholipid group, glycophosphatidylinositol, phosphatidylserine, phosphatidylethanolamine, sphingomyelin, phoshpatidylcholine, cardiolipin, phosphatidylinositol, phosphatidic acid, lysophosphoglyceride, and a cholesterol group. Preferably, the lipidated derivative is a fatty acid derivative in which the polypeptide or peptidomimetics described herein comprises a fatty acid molecule. The fatty acid molecule can be any C8-C20 fatty acid. The fatty acid molecule can be, e.g., lauric acid, palmitic acid, myristic acid, stearic acid, oleic acid, linoleic acid, linolenic acid, arachidonic acid, timnodoinc acid, erucic acid, or arachidic acid. The fatty acid may optionally contain additional functional groups, e.g., one or more amino groups on any of the carbon atoms. The fatty acid molecule can be attached to any suitable part of the inventive polypeptide or peptidomimetics. For instance, the inventive polypeptide comprises a fatty acid molecule at the amino terminus, the carboxyl terminus, or both the amino and carboxyl termini. The fatty acid molecule can be attached to the inventive polypeptide or peptidomimetics directly or through a linker.

The polypeptides and peptidomimetics according to the invention, including fatty acid derivatives thereof, can be a monomer peptide, or can be a dimmer or multimer peptide.

The polypeptides of the invention can be obtained by methods known in the art (See, for instance, Chan et al., Fmoc Solid Phase Peptide Synthesis, Oxford University Press, Oxford, United Kingdom , 2005 ; Reid, R., Peptide and Protein Drug Analysis, Marcel Dekker Company, 2000; and U.S. Patent No. 5,449,752). Also polypeptides can be recombinantly produced using the nucleic acids described herein using standard recombinant methods (See, for instance, Sambrook et al., Molecular Cloning: A Laboratory Manual 3rd ed., Cold Spring Harbor Press, Cold Spring Harbor, NY 2001). Further, some of the polypeptides of the invention can be isolated and/or purified from a source, such as a plant, a bacterium, an insect, a mammal, e.g., a rat, a human, etc. Methods of isolation and purification are well-known in the art. Alternatively, the polypeptides described herein (including functional fragments and functional variants thereof) can be synthesized or obtained from commercial companies.

Polynucleotide

The present invention also provides isolated polynucleotides encoding any one of said polypeptide of the present invention. Said polynucleotide comprises the polypeptide coding sequence that encodes any one of the present invention polypeptides, e.g., the nucleotide sequence of SEQ ID NO: 7 encoding SEQ ID NO: 1, and any degenerate variant of the coding sequence; alternatively, the polynucleotide can also include additional coding sequence and/or non-coding sequence. In another aspect, the present invention also provides isolated polynucleotides or fragments that contains a nucleotide sequence complementary to the nucleotide sequence of any one of the nucleic acid in the present invention or hybridizes with the nucleotide sequence of any one of polynucleotide under stringent condition.

Herein described are also variants of the polynucleotide, encoding same amino acid sequence of the polypeptides or polypeptide fragment, functional variant with the polypeptide (including functional fragments and functional variant). These polynucleotide variants can be naturally occurring allelic variants or non-naturally occurring variants. These nucleotide variants include substituted variants, deletion variants, and insertion variants. As known in the art, allelic variant is an alternate form of a polynucleotide, it may contain one or more nucleotide substitutions, deletions or insertions, but will not substantially alter the function of the encoded polypeptide.

The nucleic acids can be purified from natural-occurring, and also can be produced by recombination, or can be constructed based on chemical synthesis and/or enzymatic ligation reactions using procedures known in the art. For example, a nucleic acid can comprise naturally occurring nucleotides or variously modified nucleotides designed to increase the biological stability of the molecules or to increase the physical stability of the duplex formed upon hybridization (e.g., phosphorothioate derivatives and acridine substituted nucleotides). Examples of modified nucleotides that can be used to generate the nucleic acids include, but are not limited to, 5-fluorouracil, 5-bromouracil, 5-chlorouracil, 5-iodouracil, hypoxanthine, xanthine , 4-acetylcytosine , 5-(carboxyhydroxymethyl) uracil, 5-methoxyaminomethyl-2-thiouracil, uracil-5-oxyacetic acid, 5-methyl-2-thiouracil, 2-thiocytosine, 2-thiouracil, 4-thiouracil, 5-methyluracil, uracil-5-oxyacetic acid methylester, 3-(3-amino-3-N-2-carboxypropyl) uracil, and 2,6-diaminopurine and the like. Further, it can contain natural or altered linkages other than the phosphodiester bond between nucleotides, such as phosphoramidate linkages. Preferably, nucleic acids used in this invention are produced through recombination.

Recombinant Expression Vector

The invention further provides any recombinant expression vector containing the inventive polynucleotide. The recombinant expression vector of the invention can be any suitable recombinant expression vector, and can be used to transform or transfect any suitable host. Suitable vectors include those designed for propagation and expansion or for expression or both , such as plasmids and viruses. The vector can be selected from the group consisting of the pUC series, the pcDNA series, the pBluescript series, the pET series, the pGEX series, and the pEX series. Bacteriophage vectors, such as λGT10, λGTI11, λZapII, λEMBL4, etc. also can be used. Examples of plant expression vectors include pBI01, pBI101.2, pBI101.3, pBI121 and pBIN19. Examples of animal expression vectors include pEUK-Cl, pMAM and pMAMneo. Preferably, the recombinant expression vector is pcDNA series.

The recombinant expression vectors of the invention can be prepared using standard recombinant DNA techniques. Constructs of expression vectors, which are circular or linear, can be prepared to contain a replication system functional in a prokaryotic or eukaryotic host cell. Desirably, the recombinant expression vector comprises regulatory sequences, such as transcription and translation initiation and termination codons, which are specific to the type of host (e.g. , bacterium, fungus, plant , or animal) into which the vector is to be introduced, as appropriate and taking into consideration whether the vector is DNA- or RNA- based.

The recombinant expression vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the inventive expression vectors include , for instance, neomycin/G418 resistance genes, hygromycin resistance genes , histidinol resistance genes , tetracycline resistance genes, and ampicillin resistance genes.

The recombinant expression vector can comprise a native or normative promoter. The selection of promoters , e.g. , strong, weak, inducible, tissue-specific and developmental-specific, is within the ordinary skill of the artisan. Similarly, the combining of a nucleotide sequence with a promoter is also within the skill of the artisan. The promoter can be a non-viral promoter or a viral promoter , e.g., a cytomegalovirus (CMV) promoter, an SV40 promoter , an RSV promoter , and a promoter found in the long-terminal repeat of the murine stem cell virus. The inventive recombinant expression vectors can be designed for either transient expression, for stable expression , or for both. Also, the recombinant expression vectors can be made for constitutive expression or for inducible expression.

Further , the recombinant expression vectors can be made to include a suicide gene. The term "suicide gene" refers to a gene that causes the cell expressing the suicide gene to die. The suicide gene can be a gene that confers sensitivity to an agent, e.g., a drug ,upon the cell in which the gene is expressed , and causes the cell to die. Suicide

genes are known in the art (see, for example, Suicide Gene Therapy: Methods and Reviews, Springer, Caroline J. (Cancer Research UK Centre for Cancer Therapeutics at the Institute of Cancer Research, Sutton, Surrey, UK), Humana Press , 2004) and include, for example, the Herpes Simplex Virus (HSV) thymidine kinase (TK) gene , cytosine daminase , purine nucleoside phosphorylase, and nitroreductase.

Host Cell

The invention farther provides a host cell comprising any of the recombinant expression vectors described herein. As used herein, the term "host cell" refers to any type of cell that can contain the inventive recombinant expression vector. The host cell can be a eukaryotic cell, e.g. , plant, animal ,fungi ,or algae, or can be a prokaryotic cell, e.g. ,bacteria or protozoa. The host cell can be a cultured cell or a primary cell, i.e. , isolated directly from an organism, e.g., a human. The host cell can be an adherent cell or a suspended cell, i.e. , a cell that grows in suspension. Suitable host cells are known in the art and include, for instance, DH5a E. coli cells, Chinese hamster ovarian cells , monkey VERO cells, COS cells, HEK293 cells, and the like. For purposes of amplifying or replicating the recombinant expression vector, the host cell is preferably a prokaryotic cell , e.g., a DH5$\alpha$ cell. For purposes of producing a recombinant modified TCR, polypeptide, or protein, the host cell is preferably a mammalian cell. Most preferably, the host cell is a human cell. The host cell can be of any cell type , can originate from any type of tissue , and can be of any developmental stage.

Also provided by the invention is a population of cells comprising at least one host cell described herein. The population of cells can be a heterogeneous population comprising the host cell comprising any of the recombinant expression vectors described, in addition to at least one other cell , e.g., a host cell (e.g., a T cell), which does not comprise any of the recombinant expression vectors, or a cell other than a T cell, e.g. ,a B cell, a macrophage, a neutrophil, an erythrocyte, a hepatocyte, an endothelial cell, an epithelial cells, a muscle cell, a brain cell, etc. Alternatively, the population of cells can be a substantially homogeneous population, in which the population comprises mainly of host cells (e.g., consisting essentially of) comprising the recombinant expression vector. The population also can be a clonal population of cells ,in which all cells of the population are clones of a single host cell comprising a recombinant expression vector, such that all cells of the population comprise the recombinant expression vector. In one embodiment of the invention, the population of cells is a clonal population comprising host cells comprising a recombinant expression vector as described herein.

Conjugate

Included in the scope of the invention are conjugates, e.g. , bioconjugates, comprising any of the inventive polypeptides (including any of the functional fragments or functional variants) or peptidomimetics , nucleic acids, recombinant expression vectors , or host cells. Conjugates, as well as methods of synthesizing conjugates in general, are known in the art (See, for instance, Hudecz, F., Methods Mol Biol. 298: 209-223 (2005) and Kirin et al., Inorg Chem. 44(15): 5405-5415 (2005)).

Pharmaceutical Composition

The inventive polypeptides (including functional fragments and functional variants), peptidomimetics, fatty acid derivatives, conjugates, nucleic acids , recombinant expression vectors, and host cells (including populations thereof), all of which are collectively referred to as "inventive materials" hereinafter, can be isolated, purified, synthesized and/or recombinated.

The inventive materials also can be formulated into a composition, such as a pharmaceutical composition. In this regard , the invention provides a pharmaceutical composition comprising any of the polypeptides (including functional fragments and functional variants), peptidomimetics, fatty acid derivatives, conjugates, nucleic acids , recombinant expression vectors, and host cells (including populations thereof), and a pharmaceutically acceptable carrier. The inventive pharmaceutical compositions containing any of the inventive materials can comprise more than one inventive material, e.g., a polypeptide and a nucleic acid, or two or more different polypeptides. Alternatively , the pharmaceutical composition can comprise an inventive material in combination with another pharmaceutically active agent or drag , such as a chemotherapeutic agent , e.g., asparaginase, busulfan, carboplatin, cisplatin, damiorabicin, doxorubicin, fluorouracil, gemcitabine, hydroxyurea, methotrexate, paclitaxel , rituximab, vinblastine, vincristine, etc..

In a preferred embodiment of the invention , the pharmaceutical composition comprises the inventive material in combination with a lipid. The lipid can be any lipid , including, for example, a fatty acid, a phospholipid , a sterol , a sphingolipid, a terpene, a glycerolipid, a glyeerophospholipid , a prenol lipid, a saccharolipid, and a polyketide. Such lipids are known in the art.

With respect to pharmaceutical compositions, the pharmaceutically acceptable carrier can be any of those conventionally used and is limited only by chemico-physical considerations, such as solubility and lack of reactivity with

the active compound(s), and by the route of administration. The pharmaceutically acceptable carriers described herein, for example, vehicles, adjuvants, excipients , and diluents , are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular inventive material , as well as by the particular method used to administer the inventive material. Accordingly, there are a variety of suitable formulations of the pharmaceutical composition of the invention. The following formulations for oral, aerosol, parenteral, subcutaneous, intravenous, intramuscular, intraarterial, intrathecal , interperitoneal, rectal ,and vaginal administration are exemplary and are in no way limiting. More than one route can be used to administer the inventive materials, and in certain instances, a particular route can provide a more immediate and more effective response than another method.

In a preferred embodiment of the invention, the pharmaceutical composition is a topical formulation. Topical formulations are well-known to those of skill in the art. Such formulations are particularly suitable in the context of the invention for application to the skin. The topical formulation of the invention can be , for instance, a cream, a lotion, an ointment , a patch , an oil , a paste , a spray , e.g. , an aerosol spray, a gel, a roll-on liquid, a solid stick, etc. Preferably, the topical formulation of the invention is a cream , a lotion, an ointment, or a patch.

Formulations suitable-for oral administration can consist of (a) liquid solutions, such as an effective amount of the inventive material dissolved in diluents, such as water, saline, or orange juice; (b) capsules, tablets, lozenges , and troches , each containing a predetermined amount of the active ingredient, as solids or granules; (c) powders; (d) suspensions in an appropriate liquid; and (e) suitable emulsions. Liquid formulations may include diluents, such as water and alcohols, for example, ethanol , benzyl alcohol, and the polyethylene alcohols, either with or without the addition of a pharmaceutically acceptable surfactant. Capsule forms can be of the ordinary hard-or soft-shelled gelatin type containing, for example, surfactants, lubricants, and inert fillers , such as lactose , sucrose , calcium phosphate, and com starch. Tablet forms can include one or more of lactose, sucrose, mannitol, corn tarch, potato starch, alginic acid, microcrystalline cellulose, acacia, gelatin, guar gum, colloidal silicon dioxide, sodium, talc , magnesium stearate, calcium stearate, zinc stearate, stearic acid, and other excipients, colorants, diluents, buffering agents, disintegrating agents, moistening agents , preservatives, flavoring agents, and other pharmacologically compatible excipients. Lozenge forms can comprise the inventive material in a flavor, usually sucrose and acacia or tragacanth , as well as pastilles comprising the inventive material in an inert base, such as gelatin and glycerin ,or sucrose and acacia , emulsions, gels, and the like containing, in addition to, such excipients as are known in the art.

The inventive material, alone or in combination with other suitable components, can be made into aerosol formulations to be administered via inhalation. These aerosol formulations can be placed into pressurized acceptable propellants, such as dichlorodifluoromethane, propane ,nitrogen, and the like. They also may be formulated as pharmaceuticals for non-pressured preparations, such as in a nebulizer or an atomizer. Such spray formulations also may be used to spray mucosa.

Formulations suitable for parenteral administration include aqueous and non-aqueous isotonic sterile injection solutions, which can contain anti-oxidants, buffers, bacteriostats ,and solutes that render the formulation isotonic with the blood of the intended recipient, and aqueous and non-aqueous sterile suspensions that can include suspending agents, solubilizers, thickening agents, stabilizers, and preservatives. The inventive material can be administered in a physiologically acceptable diluent in a pharmaceutical carrier, such as a sterile liquid or mixture of liquids , including water, saline, aqueous dextrose and related sugar solutions, an alcohol , such as ethanol or hexadecyl alcohol, a glycol , such as propylene glycol or polyethylene glycol , dimethylsulfoxide, glycerol, ketals such as 2,2-dimethyl-1,3-dioxolane-4-methanol, ethers, oils , fatty acids , fatty acid esters or glycerides , or acetylated fatty acid glycerides with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils ,which can be used in parenteral formulations include petroleum, animal, vegetable , or synthetic oils. Specific examples of oils include peanut, soybean, sesame, cottonseed, com, olive, petrolatum, and mineral. Suitable fatty acids for use in parenteral formulations include oleic acid, stearic acid, and isostearic acid.

Suitable soaps for use in parenteral formulations include fatty alkali metal, ammonium, and triethanolaxnine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates ,alkyl ,olefin, ether, and monoglyceride sulfates, and sulfosuccinates, (c) nonionic detergents such as, for example, fatty amine oxides, fatty acid alkanolamides, and polyoxyethylenepolypropylene copolymers ,(d) amphoteric detergents such as5 for example, alkyl-p-aminopropionates, and 2-alkyl-imidazoline quaternary ammonium salts, and (e) mixtures thereof.

The parenteral formulations will typically contain from about 0.5% to about 25% by weight of the inventive material in solution. Preservatives and buffers may be used. In order to minimize or eliminate irritation at the site of injection, such compositions may contain one or more nonionic surfactants having a hydrophile-lipophile balance (HLB). The quantity of surfactant in such formulations will typically range from about 5% to about 15% by weight. Suitable

surfactants include polyethylene glycol sorbitan fatty acid esters , such as sorbitan monooleate and the high molecular weight adducts of ethylene oxide with a hydrophobic base , formed by the condensation of propylene oxide with propylene glycol. The parenteral formulations can be presented in unit-dose or multi-dose sealed containers, such as ampoules and vials, and can be stored in a freeze-dried (lyophilized) condition requiring only the addition of the sterile liquid excipient, for example, water , for injections, immediately prior to use. Extemporaneous injection solutions and suspensions can be prepared from sterile powders , granules, and tablets of the kind previously described.

Injectable formulations are in accordance with the invention. The requirements for effective pharmaceutical carriers for injectable compositions are well-known to those of ordinary skill in the art. Preferably, when administering cells , e.g. , dendritic cells , the cells are administered via injection.

Additionally , the inventive materials, or compositions comprising such inventive materials, can be made into suppositories by mixing with a variety of bases , such as emulsifying bases or water-soluble bases. Formulations suitable for vaginal administration can be presented as pessaries, tampons, creams, gels, pastes, foams, or spray formulas containing, in addition to the active ingredient, such carriers as are known in the art to be appropriate.

It will be appreciated by one of skill in the art that, in addition to the above-described pharmaceutical compositions, the inventive materials of the Invention can be formulated as inclusion complexes, such as cyclodextrin inclusion complexes, or liposomes.

For purposes of the invention, the amount or dose of the inventive material administered should be sufficient to effect, e.g., a therapeutic or prophylactic response, in the subject or animal over a reasonable time frame. For example, the dose of the inventive material should be sufficient to inhibit proliferation of a diseased cell, or treat or prevent a disease (e.g., cancer, neoplasm, or psoriasis in a period of from about 2 hours or longer, e.g., 12 to 24 or more hours, from the time of administration. In certain embodiments, the time period could be even longer. The dose will be determined by the efficacy of the particular inventive material and the condition of the animal (e.g., human), as well as the body weight of the animal (e.g., human) to be treated. Many assays for determining an administered dose are known in the art. The dose of the inventive material also will be determined by the existence, nature and extent of any adverse side effects that might accompany the administration of a particular inventive material.

One of ordinary skill in the art will readily appreciate that the inventive materials of the invention can be modified in any number of ways , such that the therapeutic or prophylactic efficacy of the inventive materials is increased through the modification. For instance, the inventive materials can be conjugated either directly or indirectly through a linker to a targeting moiety. The practice of conjugating compounds , e.g., inventive materials, to targeting moieties is known in the art. See, for instance, Wadwa et al., J. Drug Targeting 3: 111 (1995) and U.S. Patent No. 5,087,616. In another embodiment, the inventive materials can be modified into a depot form, such that the manner in which the inventive materials is released into the body to which it is administered is controlled with respect to time and location within the body (see , for example, U.S. Patent No. 4,450,150). Depot forms of inventive materials can be, for example, an implantable composition comprising the inventive materials and a porous or non-porous material, such as a polymer, wherein the inventive materials is encapsulated by or diffused throughout the material and/or degradation of the non-porous material. The depot is then implanted into the desired location within the body and the inventive materials are released from the implant at a predetermined rate.

One of ordinary skill in the art will readily appreciate that the invention provides a method of preventing and inhibiting proliferation of a diseased cell. The method comprises contacting the diseased cell with any of the pharmaceutical compositions described herein in an amount effective to inhibit proliferation of the diseased cell. In a preferred embodiment of the inventive methods, the pharmaceutical composition is topically administered to the host. In another preferred embodiment, the pharmaceutical composition is administered directly to the tumor, e.g., delivered intratumorally.

The pharmaceutical compositions according to the invention, including polypeptides (including functional fragments and functional variants) and the peptidomimetics , nucleic acids , recombinant expression vectors, and/or host cells can be used in methods of preventing and inhibiting hepatitis B virus infection-induced chronic liver diseases, including hepatitis and the resulted cirrhosis and liver cancers. Ordinary skill in the art should be readily understood that the chronic liver diseases induced by hepatitis B virus according to the present invention may be present in any host. Preferably, the host is a mammal. An especially preferred mammal is the human.

The present invention will be further illustrated below with reference to the specific examples. It should be understood that these examples are only used to describe the invention but not to limit the scope of the invention. The experimental methods with no specific conditions described in the following examples are generally performed under conventional conditions, and the materials used without specific description are purchased from common chemical reagents corporation.

Examples

Example 1: Design and preparation of polypeptides

Artificial synthesis of the fragment of polypeptides anti-HBxP1#:

The polypeptides having the amino acid sequence Gly-Ser-Ala-Val-Met-Phe-Ser-Ser-Lys-Glu-Arg-Gly (SEQ ID NO: 1) (hereafter called anti-HBxP1#) are synthesized by artificial synthetic methods. The polypeptide was prepared through solid phase peptide synthesis method, and was carried out on the Apex396 Peptide Synthesizer produced by AAPPTEC Company; the synthesis was performed in accordance with the sequence of SEQ ID NO: 1, from C-terminus carboxyl terminal to N-terminus amino terminal, to synthesize the amino acid in the sealed explosion-proof glass reactor. This refers to that the first amino acid monomer added into the sequence of amino acid was Gly in the C-terminus, followed by Arg, and then Glu, until the last Ser and Gly were added in the N-terminus. The resulting peptide was obtained by repeating adding, reacting and synthesizing. The solid phase peptide synthesis was performed with conditional blocking of unreacted amino groups with acetic anhydride and activating unbounded carboxyl groups before reacting for easier purification of the resulting peptides.

The detailed synthesis cycles were shown as follow:

1) Remove of the protection: Removed the Fmoc protecting groups by treating the resin with a solution of basic solvent (piperidine);
2) Activation and coupling: The carboxyl group of the next amino acid is activated by activator. The activated monomer couples with unbounded amino group to form peptide bond. An amount of ultra concentration reagents were used in this step to drive the reaction complete. Cycle: repeat this two step reaction until the desired sequence of amino acids was obtained;
3) Wash and cleavage: The protecting groups of peptide were removed from the polyamide by cleaving the polyamide - peptide bond with protectant (TFA). The synthesis was performed from C-terminus (carboxyl terminal) to N-terminus (amino terminal). Fixed the first amino acid Gly in the C-terminus on the resin, and removed the protecting group of Gly, and then activated the carboxyl terminal of next amino acid Arg and so on, until the last amino acid was synthesized. The resulting peptide was cleaved from the resin, and purified by HPLC to obtain the 98% pure anti-HBxP1# Finally, the mass spectrum shows the molecular weight of the peptide is 1256.50Kd.

In the solid phase peptide synthesis, the elongation of peptide chain is performed on the polystyrene resin carrier. The C-terminus of synthesized peptide reacted with chloromethylated polystyrene (chlorinated benzyl ester resin) to form benzyl ester, and then added the amino acid with the protected amino group one by one in accordance with the primary structure of peptide chain to elongate the peptide chain.

As shown in the Figure 1, the artificial synthesized polypeptide anti-HBxP1# was analyzed by High Pressure Liquid chromatography (HPLC) (apply PLC Agela C18 column), and shown the purity is 98.997%.

Example 2: The anti-HBx activities of polypeptides (*in vitro*)

Two methods were used to test the anti-HBx activities of the polypeptides in the Example 1 in vitro. One was that the cDNA expressed the polypeptides in the Example 1 was constructed into the eukaryotic expression vector cDNA3.1(+) by molecular cloning technique, and then transfected into hepatoma cells to study the peptides, so as to observe the effects of studied polypeptides on inhibiting the HBx. The other one was that directly adding the synthesized polypeptides into the medium of cultured hepatoma cells, and then observe the effects of polypeptides on inhibiting the HBx.

Two cell lines were adopted in the experiment. One is constitutive expression HBx of hepatoma cell HepG2 (stable transfected hepatoma cell HepG2 expressing HBx), and the other one is constitutive expression Hepatitis B virus complete genome of hepatoma cell HepG2.2.15 (stable transfected hepatoma cell HepG2 expressing HBV complete genome). Because HBx functions to activate the transcription factor NF-κB, it is possible to detect the inhibition of HBx at the molecular level by reporter gene-based assays. Due to the role of HBx on promoting the growth and proliferation of hepatoma cells, it can test the effects of the polypeptides on the proliferation of HepG2-X cell and HepG2.2.15 cell by (3-(4,5-dimethylthiazol-2-yl)-2,5-diphenyltetrazolium bromide (MTT).

A. The construction of polypeptides eukaryotic expression vector

1. Major materials :

1) Bacterial strain: *E. coli* DH5α (purchased from Yuanhaoping (Tianjin) Biological Technology Co., Ltd),
2) Plasmids: pcDNA3.1 (+) (purchased from Invitrogen), pEGFP-C2 purchased from Invitrogen).

2. Major reagents

| Name | Source |
|---|---|
| Agar | Solarbio |
| Plasmid Extraction mini kit | Transgen |
| Ampicillin | BBI |
| Trypsin | BBI |
| Tryptone | Promega |
| Yeast extract | Promega |
| EcoRI restriction enzyme | Takara |
| XhoI restriction enzyme | Takara |
| rTaq enzyme | Takara |
| T4 DNA ligase | Takara |
| Chloroform | BBI |

3. Major solution preparation :

1) LB solution

| | |
|---|---|
| Tryptone | 2.0g |
| Yeast extract | 1.0g |
| NaCl | 2.0g |
| Add $ddH_2O$ settled to | 200ml |

$1.03 \times 10^5$ Pa, autoclave 20min.

2) LB solid medium

| | |
|---|---|
| Tryptone | 2.0g |
| Yeast extract | 1.0g |
| NaCl | 2.0g |
| Agar | 3.0g |
| Add $ddH_2O$ settled to | 200ml |

$1.03 \times 10^5$ Pa, autoclave 20min.

3) 10mg/ml Ethidium bromide (EB)

| | |
|---|---|
| EB | 0.2g |
| $ddH_2O$ | 20ml |

Working solution: 0.5ug/ml

4) TBE electrophoretic buffer

5×Stock buffer:

| | |
|---|---|
| Tris-base | 54 g |
| Boric acid | 27.5 g |
| 0.5 mol/L EDTA (pH 8.0) | 20 ml |
| Add $ddH_2O$ settled to | 1000 ml |

4. Annealing system

| Reagents | Addition (μl) |
|---|---|
| $ddH_2O$ | 20 |
| 5 × Annealing Buffer | 10 |
| Forward DNA oligo (50 μM) | 10 |
| ( 5'-AATTCATGGGAAGCGCAGTGATGTTTTCCAGTAAA GAACGCGGACGTTGCACCTGAC-3' ) (SEQ ID NO:13) | |
| Reverse DNA oligo (50 μM) | 10 |
| ( 5'-TCGAGTCAGGTGCAACGTCCGCGTTCTTTACTGGA AAACATCACTGCGCTTCCCATG-3' ) (SEQ ID NO:14) | |

5. Annealing conditions : 95°C, 2 min; decrease to 25°C at a rate of 0.1°C per 8 sec, 90min; 4°C, ∞.

6. pcDNA3.1 (+) mini-preparation of empty plasmid vector

Stored DH5α strain with pcDNA3.1 (+) plasmid was activated. Single colony was picked to add into LB medium (100mg/L ampicillin contained), and then cultured at 37 °C overnight. TransGen Plasmid Extraction mini kit was used to extract the plasmids.

7. Double digestion reaction system:

| Reagent | Addition (μl) |
|---|---|
| Restriction enzyme EcoRI | 2 |
| Restriction enzyme XhoI | 2 |
| 10 × M buffer | 4 |
| DNA | Less than 2 μg |
| ddH2O | Settled to 40 μl |

After mixed, react at 37°Cfor 3-6 hr.

8. Ligation reaction system:

| Reagent | Addition (μl) |
|---|---|
| Annealing products | 0.3 pmol |
| Plasmid double digestion extracting products | 0.03 pmol |
| 10 × ligation buffer | 2.5 μl |
| Ligase | 1 μl |
| ddH2O | Setteled to 25 μl |

After mixed, react at 16°C overnight. Synthesized base pair sequence is ggaagcgcag tgatgttttc cagtaaagaa cgcgga (SEQ ID NO:7) , and restriction map as shown in the Figure 8.

9. Transformation step

1) Thaw DH5α competent cells (purchased from TianGen, 100 μl) on ice;
2) Put all the 25 μl ligation products in the Step 8 into 1.5ml tubes that have the competent cells, and gently mixed cells with the pipet tip. Incubated the tubes on ice for 30 min;
3) Heat shock at 42°C for exactly 90 sec ;
4) Placed tubes on ice for 2 min immediately;
5) Added 500 μl LB without containing antibiotics, and then pre-culture at 37°C for 45 min;
6) Spread 100 μl bacteria solutions onto LB plates with 100mg/L ampicillin (appropriate adjust the amount according to the intensity of colonies), then allowed plates to dry and incubate inverted at 37°C overnight;
7) After single clear colonies grew on the plates, picked up a single colony to add into LB medium, and then cultured at 37°C overnight.

10. Colony PCR reaction system

| Reagents | Addition(μl) |
|---|---|
| $ddH_2O$ | 35.75 |
| 10 × buffer | 5 |
| dNTP (10mmol/L) | 4 |
| Forward Primer (20 μM) | 1 |
| 5'-TAATACGACTCACTATAGGG-3' (SEQ ID NO: 15) | |
| Reverse Primer (20 μM) | 1 |
| 5'-TAGAAGGCACAGTCGAGG-3' (SEQ ID NO:16) | |
| Template DNA (bacteria solution) | 3 |
| Taq enzyme | 0.25 |

11. Colony PCR reaction conditions:

| | | |
|---|---|---|
| 94℃ | 10 min | |
| 94℃ | 30 sec | 35 cycles |
| 53℃ | 30 sec | |
| 72℃ | 15 sec | |
| 72℃ | 10 min | |
| 4℃ | ∞ | |

After reaction, 1.5% agarose gel electrophoresis was undertaken to analyze the PCR products. The positive clones were sequenced by biotech Corporation. And the plasmid was named p-Anti-HBxP1#.

B. *In vitro* effective experiments

1. Cell line:

Table 1 Cell lines applied in the experiments

| Cell line | Property, usage and description | Source |
|---|---|---|
| HepG2 | Hepatoma cell line | Shanghai Jinma Biological Technology Co., Ltd |
| HepG2-X | Stable transfected HepG2 cell expressing HBx<br>Wang Q, et al. Neoplasia. 2010;12 (2): 103 -15. | for our own construction and preservation |
| HepG2.2.15 | Stable transfected HepG2 cell expressing HBV complete genome | Shanghai Jinma Biological Technology Co., Ltd |
| L-O2 | Immortalized normal hepatic cells | Nanjing Kaiji Biotechnology Development Co., Ltd |
| L-O2-X | Stable transfected L-O2 cell expressing HBx<br>Zhang WY, et al. Acta Pharmacol Sinica. 2009; 30(8): 1153-61. | for our own construction and preservation |

2. Major reagents

| Reagents | Source |
|---|---|
| RPMI1640 medium | Gibco |
| DMEM medium | Gibco |
| Lipofectamine 2000 | Invitrogen |
| Mycillin | Solarbio |
| Trypsin | BBI |
| Fetal bovine serum | Hyclone |

3. Dual-Luciferase reporter gene analysis

(1) Plated the cells at exponential phase (the cells in the above Table 1) at a density 0.75× $10^5$ cells/ml in a 24-well plate with 500μl per well. Ideally cells should be 90% confluent prior to transfection;
(2) Reporter gene vectors containing promoter(pGL3-NF-κB ,0.3μg, purchased from Yuanhaoping (Tianjin) Biological Technology Co., Ltd) were transfected into cells by using lipofectamine transfection reagent, and Renilla luciferase expressing vectors (pRL-TK, 0.1μg, purchased from Promega) were transfected as controls. Simultaneously, different amount of polypeptides plasmids from Step A (0.25μg, 0.5μg and 0.75μg) or different concentrations of synthesized polypeptides from Example 1 (0.1μM, 1μM, 10μM and 100μM) were added, and repeated each concentration in three wells;
(3) After transfection for 48 hr (or after incubation with artificial synthesized polypeptide Anti-HBxP1# for 24 hr), washed the cells with PBS for three times;
(4) Added 100 μl 1x Passive Lysis Buffer (PLB) into transfected cells per well, and lysed the cell at room temperature for 15 min. Transferred the cell lysate to a 1.5 ml Eppendorf (EP) tube;
(5) Centrifuged at 12,000 rpm for 30 min, and transfered the supernatant to a fresh EP tube;
(6) Added an aliquot of cell lysate into each EP tube with 100 μl Luciferase Assay Buffer II (LARII), and mixed well;
(7) Immediately put the EP tubes into a Luminometer (manufactured by Turner Biosystems). Within 10 sec after 2 sec balance, measured the luminescence.
(8) Added 100 μl fluorescence bleacher. Firefly luciferase was quenched, while Renilla luciferase started to react.
(9) Measured the luminescence within 10 sec.

The relative activity was the ratio of the value of the first luminescence to the value of the second luminescence.

Results shown are representative of three independent experiments according to Mean±SD, and statistical significance was calculated using Student's *t* test.

As shown in the Figure 2, p-Anti-HBxP1# inhibits the activity of NF-κB promoter in the HepG2-X cells, L-O2-X cells and HepG2.2.15 cells in a dose-dependent manner, but has no effect on the hepatoma cells HepG2 and hepatoma cells L-O2 which do not express HBx. The experiments further indicated that the plasmids containing genes encoding 5 variants of Anti-HBxP1# also inhibit the activity of NF-κB promoter in the HepG2-X cells and L-O2-X cells. Student's *t*-test is employed for statistical analysis, * $P<0.05$, ** $P<0.01$.

As shown in the Figure 3, incubation with artificial synthesized polypeptide Anti-HBxP1# inhibits the activity of NF-κB promoter in the HepG2-X cells, L-O2-X cells and HepG2.2.15 cells in a dose-dependent manner, but has no effect on the hepatoma cells HepG2 and hepatoma cells L-O2 which do not express HBx. Student's *t*-test is employed for statistical analysis, * $P<0.05$, ** $P<0.01$.

4. MTT assay

(1) Plate the cells: suspended the cells at exponential phase (the cells in the above Table 1) in RPMI1640 or DMEM medium with 10% FBS, and plated 4000-5000 cells in a 96-well plate with 100μl per well;
Culture the cells: Within 12 hr for adhesion of cultured cells, different amount of polypeptides plasmids from Step A (0.25μg, 0.5μg and 0.75μg) or different concentrations of synthesized polypeptides from Example 1 (0.1 μM, 1μM, 10μM and 100μM) were transfected with 8 wells for each concentration, and were incubated for 48 hr in the same condition. 0.3μg pEGFP-C2 plasmids and 1μg p-Anti-HBxP1# were cotransfected, and using a fluorescence microscope to check for cells containing the GFP plasmid after 24 hr to make sure the percentage of cells that contain GFP is over 70%;
(2) Coloration: added 20μl MTT solution (5 mg/ml MTT in PBS buffer (pH7.4)) for each well;
(3) Incubated for 4 hr, and carefully aspirate off the supernatant from the wells. Added 150μl DMSO into each well, shaking for 10 min until the crystals dissolved;
(4) Comparison: Read in an ELISA reader at 490 nm to measure the absorbance for each well. Results were calculated by using Student's *t*-test.

As shown in the Figure 4, Anti-HBxP1# inhibits the activity of NF-κB promoter in the HepG2-X cells, L-O2-X cells and HepG2.2.15 cells in a dose-dependent manner, and has no effect on the hepatoma cells HepG2 and hepatoma cells L-O2 which do not express HBx. Similarly, the genes containing 5 variants of Anti-HBxP1# inhibit the activity of NF-κB promoter in the HepG2-X cells and L-O2-X cells. Student's *t*-test was employed for statistical analysis, * $P<0.05$,

** P<0.01.

As shown in the Figure 5, MTT assay reveals that artificial synthesized polypeptide Anti-HBxP1# inhibits the activity of NF-κB promoter in the HepG2-X cells, L-O2-X cells and HepG2.2.15 cells in a dose-dependent manner, but has no effect on the hepatoma cells HepG2 and hepatoma cells L-O2 which do not express HBx. Student's t-test was employed for statistical analysis, * P<0.05, ** P<0.01.

Example 3: The anti-HBx activities of polypeptides *in vivo*

Suspended the HepG2-X or HepG2.2.15 cells at exponential phase by treating with trypsin, and counted the number of cells to dilute to 1 x $10^7$ cells/ml with physiological saline, and then store in the ice water. 12 Mice used were 4- to 6-week-old BALB/C females, and were randomly divided into two groups: ① Control group, injected 0.2ml diluted cells to the armpit of left forelimb for each mouse, and only injected 0.5ml $ddH_2O$ (without polypeptide drugs); ② experimental group (the treatment dose was 10 mg/kg), injected 0.2ml diluted cells to the armpit of left forelimb for each mouse, and within 7 days after the injection, tumor volume ($V=L \times W^2 \times 0.5$) reached to 100 $mm^3$. And then, injected the above mentioned polypeptide drugs (dried polypeptide drug solved in 0.5ml $ddH_2O$) once in two day, total 10 times, and recorded the tumor volume before injection. Within 24 hr after the last dose, weighted the mice, and killed the mice to weight the tumor tissue, anti-tumor rate was calculated as follow:

$$\text{anti-tumor rate} = \frac{\text{The average tumor volume of control- experiment group}}{\text{The average tumor volume of control}} \times 100\%$$

As shown in Table 1, 2 and Figure 6, 7, the results of animal experiment indicate that artificial synthesized polypeptide Anti-HBxP1# inhibits the growth and proliferation in the HepG2-X cells and HepG2.2.15 cells. Student's *t*-test was employed for statistical analysis, ** P<0.01.

Table 1. The effect of artificial synthesized polypeptide Anti-HBxP1# in the HepG2-X cells:

| Group | Before dose/After dose | Weight (X±S, g) | | Tumor weight (X±S, g) | Anti-tumor rate (%) |
|---|---|---|---|---|---|
| | | Before dose | After dose | | |
| Control | 6/6 | 15.25±0.61 | 21.50±0.84 | 0.93±0.12 | - |
| Experimental | 6/6 | 15.75±0.99 | 21.08±0.74 | 0.37±0.14 | 60.22 |

Table 2. The effect of artificial synthesized polypeptide Anti-HBxP1# in the HepG2.2.15 cells:

| Group | Before dose/After dose | Weight (X±S, g) | | Tumor weight (X±S, g) | Anti-tumor rate (%) |
|---|---|---|---|---|---|
| | | Before dose | After dose | | |
| Control | 6/6 | 15.08±0.92 | 20.92±0.85 | 0.63±0.17 | - |
| Experimental | 6/6 | 15.17±1.17 | 21.08±0.81 | 0.37±0.19 | 41.27 |

Example 4: The functional fragments and functional variants

The invention also provides the role of the functional fragments and functional variants. The sequences in the table below are obtained by adding amino acids (e.g., add an amino acid in the terminal) or replacing a conserved amino acid (e.g., replace an amino acid with the same type of an amino acid) on basis of anti-HBxP1#. Artificially synthesized the polypeptide fragments according to the sequences (method as above), and then, used the above-mentioned gene reporter and MTT assay, and observe the change of the role of the polypeptides.

| Synthetic polypeptide fragments | Amino acid sequence | SEQ ID NO. |
|---|---|---|
| anti-HBxP1# | Gly-Ser-Ala-Val-Met-Phe-Ser-Ser-Lys-Glu-Arg-Gly or G-S-A-V-M-F-S-S-K-E-R-G | 1 |
| anti-HBxP1#-1 | Ile-Gly-Ser-Ala-Val-Met-Phe-Ser-Ser-Lys-Glu-Arg-Gly or I-G-S-A-V-M-F-S-S-K-E-R-G | 2 |
| anti-HBxP1#-2 | Val-Gly-Ser-Ala-Val-Met-Phe-Ser-Ser-Lys-Glu-Arg-Gly orV-G-S-A-V-M-F-S-S-K-E-R-G | 3 |
| anti-HBxP1#-3 | Gly-Ser-Ala-Val-Met-Phe-Ser-Ser-Lys-Asp-Arg-Gly or G-S-A-V-M-F-S-S-K-D-R-G | 4 |
| anti-HBxP1#-4 | Gly-Ser-Ala-Val-Met-Phe-Ser-Ser-Arg-Glu-Arg-Gly or G-S-A-V-M-F-S-S-R-E-R-G | 5 |
| anti-HBxP1#-5 | Gly-Ser-Ala-Val-Met-Phe-Ser-Ser-Lys-Glu-His-Gly or G-S-A-V-M-F-S-S-K-E-H-G | 6 |
| Synthetic polypeptide fragments | Nuclotide sequence | SEQ ID NO. |
| anti-HBxP1# | ggaagcgcag tgatgttttc cagtaaagaa cgcgga | 7 |
| anti-HBxP1#-1 | atcggaagcg cagtgatgtt ttccagtaaa gaacgcgga | 8 |
| anti-HBxP1#-2 | gtgggaagcg cagtgatgtt ttccagtaaa gaacgcgga | 9 |
| anti-HBxP1#-3 | ggaagcgcag tgatgttttc cagtaaagac cgcgga | 10 |
| anti-HBxP1#-4 | ggaagcgcag tgatgttttc cagtcgcgaa cgcgga | 11 |
| anti-HBxP1#-5 | ggaagcgcag tgatgttttc cagtaaagaa cacgga | 12 |

As shown in the Figure 2, the genes encoding 5 variants of Anti-HBxP1# inhibit the activity of NF-κB promoter in the HepG2-X cells, L-O2-X cells and HepG2.2.15 cells. Student's t-test was employed for statistical analysis, * $P<0.05$, ** $P<0.01$.

As shown in the Figure 4, the genes encoding 5 variants of Anti-HBxP1# inhibit the cell growth and proliferation in the HepG2-X cells, L-O2-X cells and HepG2.2.15 cells. Student's t-test was employed for statistical analysis, * $P<0.05$, ** $P<0.01$.

Example 5: Polypeptide drugs acute toxicity test

Control group and experimental group contained 10 Kunming white mice, with 5 females and 5 males, respectively. Experimental group injected Anti-HBxP1# with the concentration of 1g/kg (2.5mg dried polypeptide drug solved in 0.124ml $ddH_2O$) by tail intravenous injection; control group, injected 0.25ml $ddH_2O$. Consecutively observed the mice for 24 hr after injection.

The polypeptide drugs acute toxicity test showed that the mice do not have abnormal behaviors, and no abnormal changes in weight compared with control group.

The use of any and all examples, or exemplary language (e.g., "such as") provided herein, is intended merely to better illuminate the invention and does not pose a limitation on the scope of the invention unless otherwise claimed. Preferred embodiments of this invention are described herein, including the best mode known to the inventors for carrying

out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. The invention includes all modifications and equivalents of the subject matter recited in the claims appended hereto as permitted by applicable law unless otherwise indicated herein or otherwise clearly contradicted by context.

## Claims

1. An isolated polypeptide or peptidomimetic consisting of an amino acid sequence as shown in SEQ ID NO: 1 or consisting of an amino acid sequence having at least 70% preferably at least 80%, more preferably at least 90% identity with the amino acid sequence as shown in SEQ ID NO:1, said polypeptide or peptidomimetic having an effect of inhibiting HBx protein and the occurrence and development of chronic liver diseases resulting from the hepatitis B virus infection.

2. The polypeptide or peptidomimetic according to claim 1, wherein said chronic liver diseases resulted from hepatitis B virus infection comprise hepatitis, cirrhosis, and liver cancer.

3. An isolated polypeptide or peptidomimetic consisting of an amino acid sequence obtained from conservative substitution of one amino acid of SEQ ID NO:1, or addition of one amino acid at the terminus of SEQ ID NO:1.

4. An isolated polypeptide or peptidomimetic comprising any one of the amino acid sequences as shown in SEQ ID NOs: 2 to 6.

5. An isolated polynucleotide consisting of a polynucleotide selected from the group consisting of:

   a) the polynucleotide encoding the amino acid sequence as shown in SEQ ID NO: 1; and
   b) a polynucleotide that is complementary to the polynucleotide of a) or that is capable of hybridizing with the polynucleotide of a) under stringent condition.

6. An isolated polynucleotide comprising a polynucleotide selected from the group consisting of:

   a) the polynucleotide encoding any one of the amino acid sequences as shown in SEQ ID NOs: 2 to 6; and
   b) a polynucleotide that is complementary to the polynucleotide of a) or that is capable of hybridizing with the polynucleotide of a) under stringent condition.

7. A recombination expression vector comprising the polynucleotide of claim 5 or 6.

8. A host cell including the recombination expression vector of claim 7.

9. The polypeptide according to any one of claims 1-4 for use in treating and preventing chronic liver diseases resulting from the hepatitis B virus infection.

10. The polynucleotide according to any one of claims 5-6 for use in treating and preventing chronic liver disease resulting from the hepatitis B virus infection.

11. The recombination expression vector according to claim 7 for use in treating and preventing chronic liver disease resulting from the hepatitis B virus infection.

12. A therapeutic vaccine comprising the polypeptide of claim 1-4, the polynucleotide of claim 5-6, or the recombinant expression vector of claim 7 for treating and preventing hepatitis B.

13. A pharmaceutical composition, comprising any one of the polypeptides or peptidomimetic according to claims 1-4, and optional pharmaceutical carriers.

14. A pharmaceutical composition, comprising any one of the polynucleotides according to any one of claims 5-6, and optional pharmaceutical carriers.

15. A pharmaceutical composition, comprising the recombination expression vector according to claim 7, and optional

pharmaceutical carriers.

**Patentansprüche**

1. Isoliertes Polypeptid oder Peptidomimetikum, das aus einer wie in SEQ ID NO: 1 dargestellten Aminosäuresequenz besteht oder das aus einer Aminosäuresequenz besteht, die mindestens 70 %, bevorzugt mindestens 80 %, bevorzugter mindestens 90 % Identität mit der wie in SEQ ID NO: 1 dargestellten Aminosäuresequenz aufweist, wobei das Polypeptid oder Peptidomimetikum einen Effekt hat, das HBx-Protein und das Auftreten sowie die Entstehung von chronischen Lebererkrankungen, die durch die Hepatitis-B-Virusinfektion entstehen, zu inhibieren.

2. Polypeptid oder Peptidomimetikum nach Anspruch 1, wobei die durch Hepatitis-B-Virusinfektion entstandenen chronischen Lebererkrankungen Hepatitis, Leberzirrhose und Leberkrebs umfassen.

3. Isoliertes Polypeptid oder Peptidomimetikum, das aus einer Aminosäuresequenz besteht, die durch konservative Substitution einer Aminosäure von SEQ ID NO: 1, oder Hinzufügen einer Aminosäure an dem Terminus von SEQ ID NO: 1, erhalten wird.

4. Isoliertes Polypeptid oder Peptidomimetikum, das eine der wie in SEQ ID NO: 2 bis 6 dargestellten Aminosäuresequenzen umfasst.

5. Isoliertes Polynukleotid, das aus einem Polynukleotid besteht, ausgewählt aus der Gruppe, bestehend aus:

   a) dem Polynukleotid, das die wie in SEQ ID NO: 1 dargestellte Aminosäuresequenz kodiert; und
   b) einem Polynukleotid, das komplementär zu dem Polynukleotid aus a) ist, oder das fähig ist, mit dem Polynukleotid aus a) unter stringenter Bedingung zu hybridisieren.

6. Isoliertes Polynukleotid, das ein Polynukleotid umfasst, ausgewählt aus der Gruppe, bestehend aus:

   a) dem Polynukleotid,, das eine der wie in SEQ ID NO: 2 bis 6 dargestellten Aminosäuresequenzen kodiert; und
   b) einem Polynukleotid, das komplementär zu dem Polynukleotid aus a) ist, oder das fähig ist, mit dem Polynukleotid aus a) unter stringenter Bedingung zu hybridisieren.

7. Rekombinationsexpressionsvektor, der das Polynukleotid nach Anspruch 5 oder 6 umfasst.

8. Wirtszelle, die den Rekombinationsexpressionsvektor nach Anspruch 7 beinhaltet.

9. Polypeptid nach einem der Ansprüche 1-4 zur Verwendung beim Behandeln und Verhindern chronischer Lebererkrankungen, die durch die Hepatitis-B-Virusinfektion entstehen.

10. Polynukleotid nach einem der Ansprüche 5-6 zur Verwendung beim Behandeln und Verhindern chronischer Lebererkrankungen, die durch die Hepatitis-B-Virusinfektion entstehen.

11. Rekombinationsexpressionsvektor nach Anspruch 7 zur Verwendung beim Behandeln und Verhindern chronischer Lebererkrankungen, die durch die Hepatitis-B-Virusinfektion entstehen.

12. Therapeutischer Impfstoff, der das Polypeptid nach Anspruch 1-4, das Polynukleotid nach Anspruch 5-6 oder den rekombinanten Expressionsvektor nach Anspruch 7 umfasst, zum Behandeln und Verhindern von Hepatitis B.

13. Pharmazeutische Zusammensetzung, die eines der Polypeptide oder Peptidomimetika nach Ansprüchen 1-4, und wahlweise pharmazeutische Träger, umfasst.

14. Pharmazeutische Zusammensetzung, die eines der Polynukleotide nach einem der Ansprüche 5-6, und wahlweise pharmazeutische Träger, umfasst.

15. Pharmazeutische Zusammensetzung, die den Rekombinationsexpressionsvektor nach Anspruch 7, und wahlweise pharmazeutische Träger, umfasst.

## Revendications

1. Polypeptide ou peptidomimétique isolé constitué d'une séquence d'acides aminés représentée par SEQ ID NO : 1 ou constitué d'une séquence d'acides aminés présentant au moins 70 %, de préférence au moins 80 %, de façon davantage préférée au moins 90 % d'identité avec la séquence d'acides aminés représentée par SEQ ID NO : 1, ledit polypeptide ou peptidomimétique présentant un effet d'inhibition de la protéine HBx et de l'occurrence et du développement de maladies hépatiques chroniques résultant de l'infection par le virus de l'hépatite B.

2. Polypeptide ou peptidomimétique selon la revendication 1, où lesdites maladies hépatiques chroniques résultant de l'infection par le virus de l'hépatite B comprennent l'hépatite, la cirrhose, et le cancer du foie.

3. Polypeptide ou peptidomimétique isolé constitué d'une séquence d'acides aminés obtenue à partir de la substitution conservatrice d'un acide aminé de SEQ ID NO : 1, de l'addition d'un acide aminé à l'extrémité de SEQ ID NO : 1.

4. Polypeptide ou peptidomimétique isolé comprenant l'une quelconque des séquences d'acides aminés représentées par SEQ ID NO : 2 à 6.

5. Polynucléotide isolé constitué d'un polynucléotide choisi dans le groupe constitué de :

   a) le polynucléotide codant pour la séquence d'acides aminés représentée par SEQ ID NO : 1 ; et
   b) un polynucléotide qui est complémentaire du polynucléotide de a) ou qui est capable de s'hybrider avec le polynucléotide de a) dans des conditions stringentes.

6. Polynucléotide isolé comprenant un polynucléotide choisi dans le groupe constitué de :

   a) le polynucléotide codant pour l'une quelconque des séquences d'acides aminés représentées par SEQ ID NO : 2 à 6 ; et
   b) un polynucléotide qui est complémentaire du polynucléotide de a) ou qui est capable de s'hybrider avec le polynucléotide de a) dans des conditions stringentes.

7. Vecteur d'expression de recombinaison comprenant le polynucléotide selon la revendication 5 ou 6.

8. Cellule hôte comprenant le vecteur d'expression de recombinaison selon la revendication 7.

9. Polypeptide selon l'une quelconque des revendications 1 à 4 pour une utilisation dans le traitement et la prévention de maladies hépatiques chroniques résultant de l'infection par le virus de l'hépatite B.

10. Polynucléotide selon l'une quelconque des revendications 5 ou 6, pour une utilisation dans le traitement et la prévention d'une maladie hépatique chronique résultant de l'infection par le virus de l'hépatite B.

11. Vecteur d'expression de recombinaison selon la revendication 7 pour une utilisation dans le traitement et la prévention d'une maladie hépatique chronique résultant de l'infection par le virus de l'hépatite B.

12. Vaccin thérapeutique comprenant le polypeptide selon les revendications 1 à 4, le polynucléotide selon les revendications 5 ou 6, ou le vecteur d'expression de recombinaison selon la revendication 7 pour le traitement et la prévention de l'hépatite B.

13. Composition pharmaceutique, comprenant l'un quelconque des polypeptides ou peptidomimétiques selon les revendications 1 à 4, et des supports pharmaceutiques éventuels.

14. Composition pharmaceutique, comprenant l'un quelconque des polynucléotides selon l'une quelconque des revendications 5 ou 6, et des supports pharmaceutiques éventuels.

15. Composition pharmaceutique, comprenant le vecteur d'expression de recombinaison selon la revendication 7, et des supports pharmaceutiques éventuels.

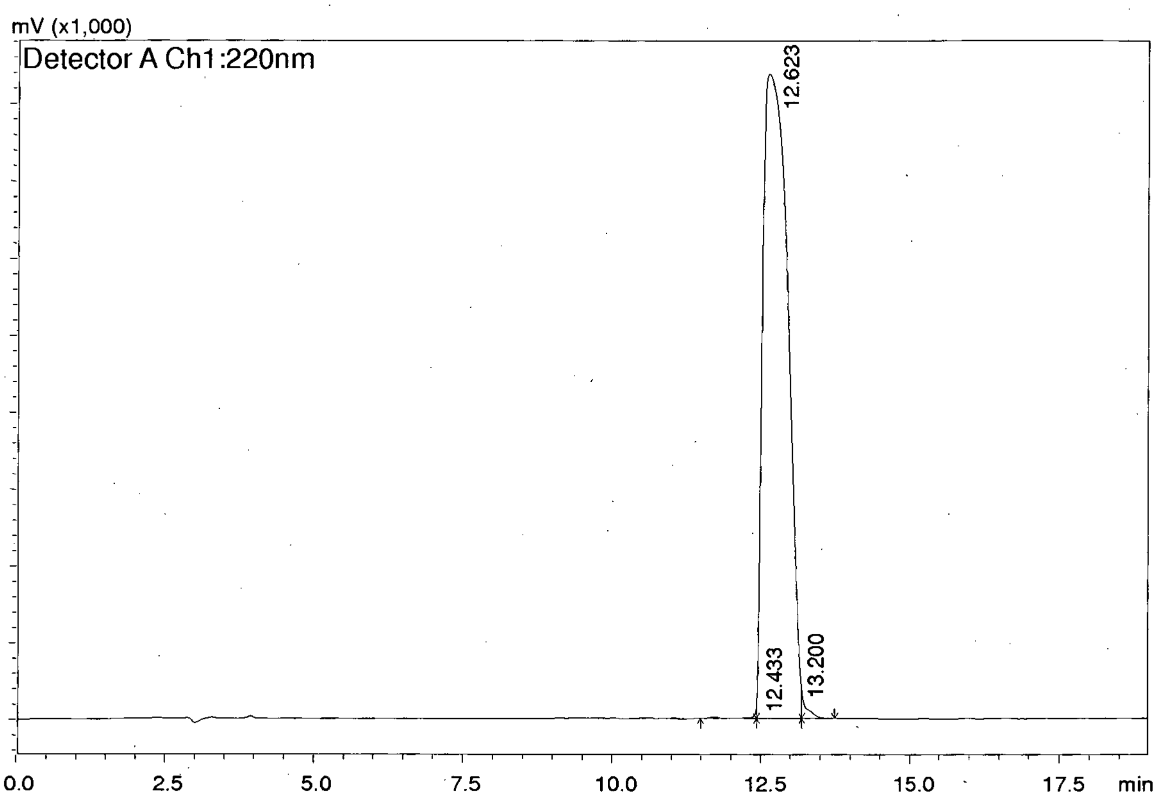
mV (x1,000)
Detector A Ch1:220nm
12.623
12.433
13.200
0.0
2.5
5.0
7.5
10.0
12.5
15.0
17.5
min

Fig 1

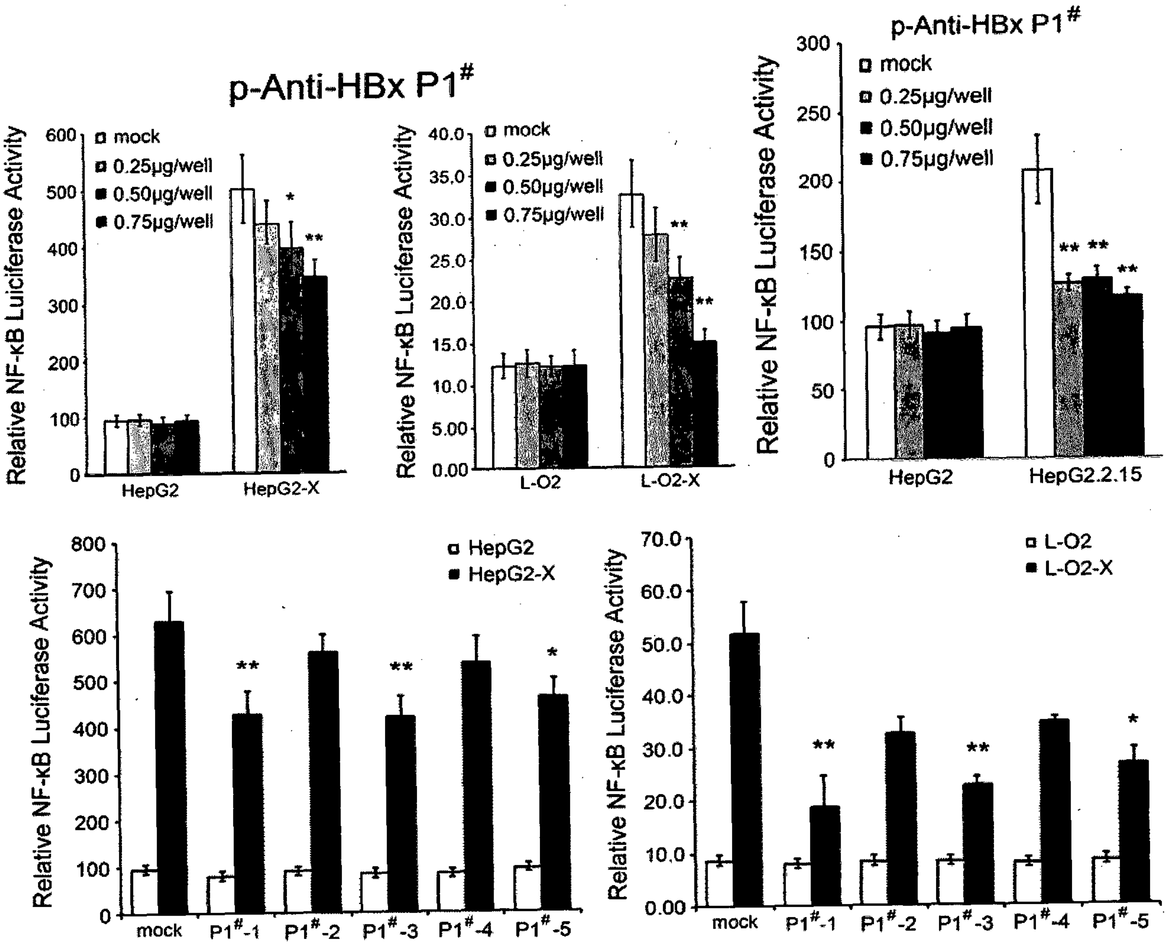
p-Anti-HBx P1#
Relative NF-κB Luciferase Activity
mock
0.25μg/well
0.50μg/well
0.75μg/well
HepG2
HepG2-X
L-O2
L-O2-X
HepG2.2.15
HepG2
HepG2-X
L-O2
L-O2-X
mock
P1#-1
P1#-2
P1#-3
P1#-4
P1#-5

Fig 2

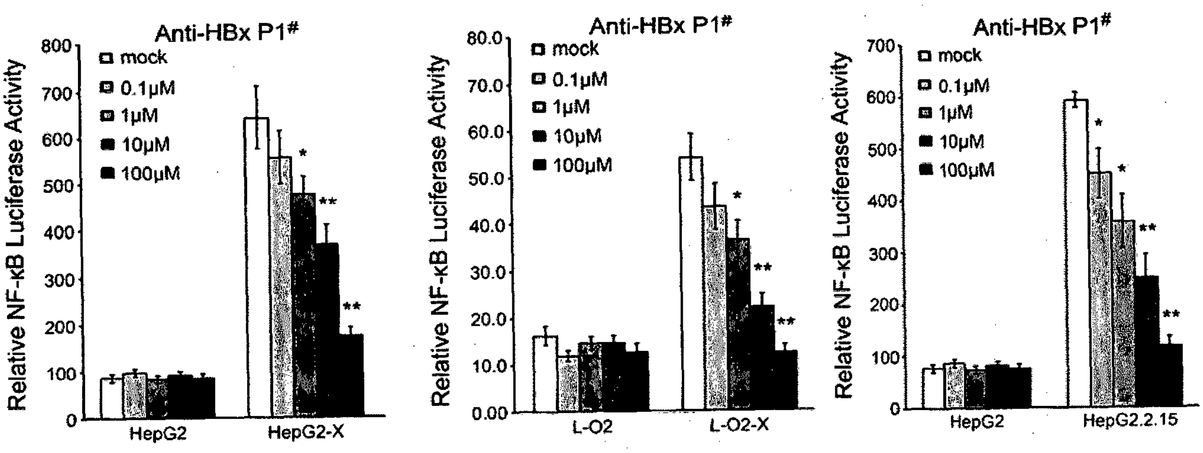
Anti-HBx P1#
Relative NF-κB Luciferase Activity
mock
0.1μM
1μM
10μM
100μM
HepG2
HepG2-X
L-O2
L-O2-X
HepG2.2.15

Fig 3

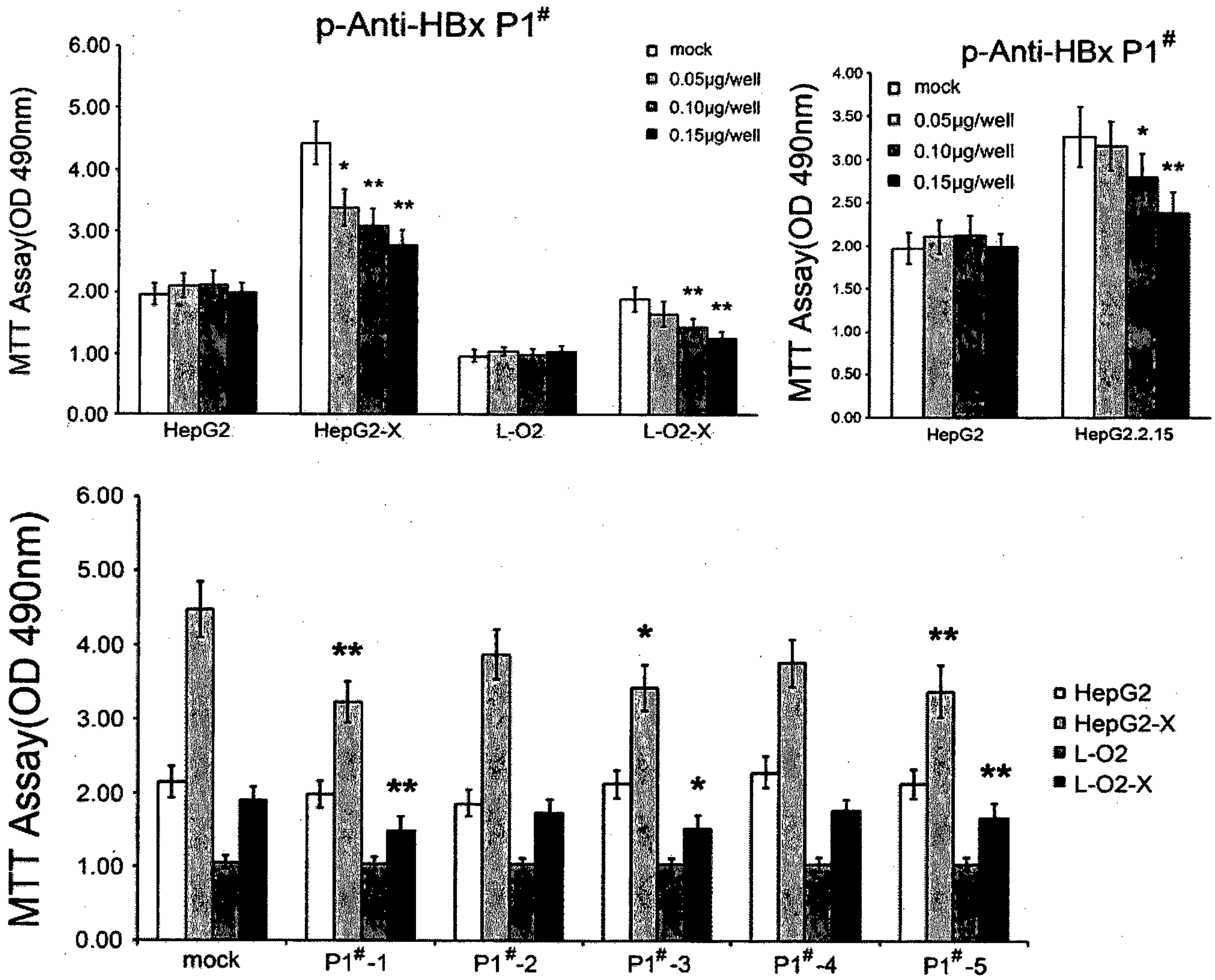
p-Anti-HBx P1#
mock
0.05μg/well
0.10μg/well
0.15μg/well
MTT Assay(OD 490nm)
6.00
5.00
4.00
3.00
2.00
1.00
0.00
HepG2
HepG2-X
L-O2
L-O2-X
p-Anti-HBx P1#
mock
0.05μg/well
0.10μg/well
0.15μg/well
MTT Assay(OD 490nm)
4.00
3.50
3.00
2.50
2.00
1.50
1.00
0.50
0.00
HepG2
HepG2.2.15
MTT Assay(OD 490nm)
6.00
5.00
4.00
3.00
2.00
1.00
0.00
mock
P1#-1
P1#-2
P1#-3
P1#-4
P1#-5
HepG2
HepG2-X
L-O2
L-O2-X

Fig 4

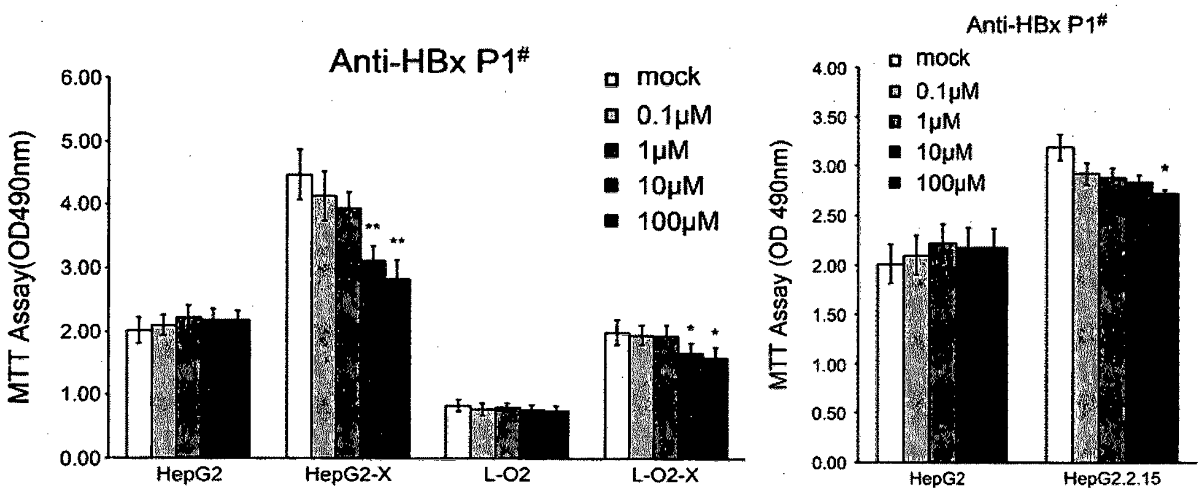
Anti-HBx P1#
mock
0.1μM
1μM
10μM
100μM
MTT Assay(OD490nm)
6.00
5.00
4.00
3.00
2.00
1.00
0.00
HepG2
HepG2-X
L-O2
L-O2-X
Anti-HBx P1#
mock
0.1μM
1μM
10μM
100μM
MTT Assay (OD 490nm)
4.00
3.50
3.00
2.50
2.00
1.50
1.00
0.50
0.00
HepG2
HepG2.2.15

Fig 5

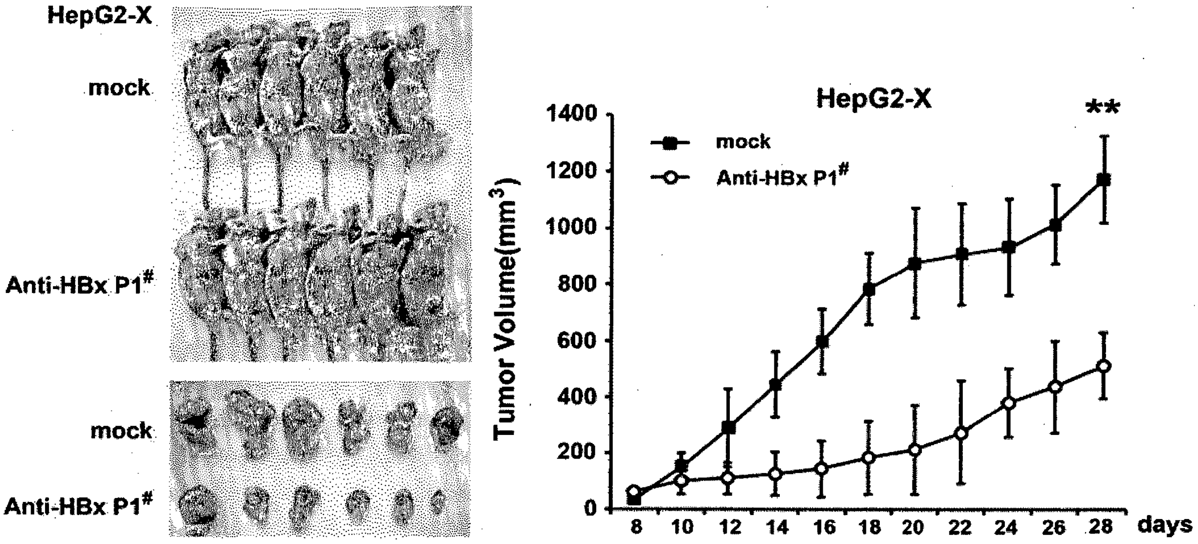
HepG2-X
mock
Anti-HBx P1#
mock
Anti-HBx P1#
HepG2-X
**
mock
Anti-HBx P1#
Tumor Volume(mm3)
1400
1200
1000
800
600
400
200
0
8
10
12
14
16
18
20
22
24
26
28
days

Fig 6

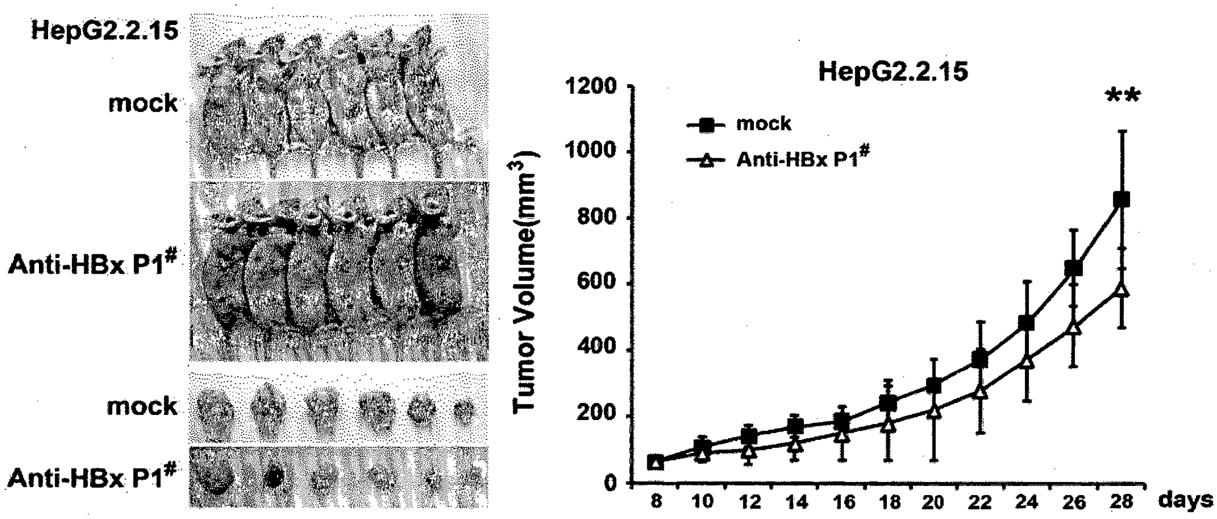
HepG2.2.15
mock
Anti-HBx P1#
mock
Anti-HBx P1#
HepG2.2.15
**
mock
Anti-HBx P1#
Tumor Volume(mm3)
1200
1000
800
600
400
200
0
8
10
12
14
16
18
20
22
24
26
28
days

Fig 7

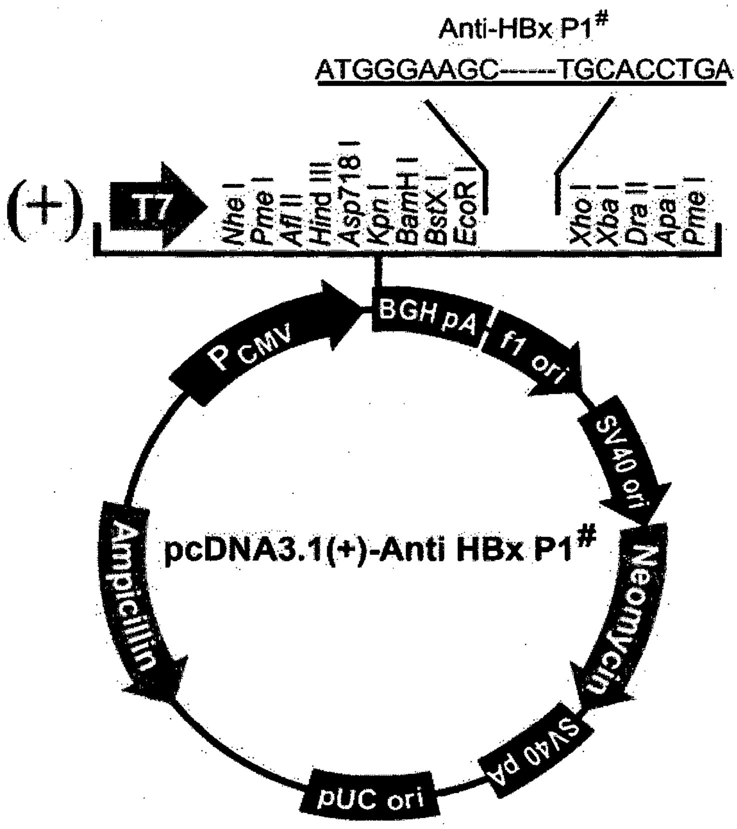
Anti-HBx P1#
ATGGGAAGC------TGCACCTGA
(+)
T7
Nhe I
Pme I
Afl II
Hind III
Asp718 I
Kpn I
BamH I
BstX I
EcoR I
Xho I
Xba I
Dra II
Apa I
Pme I
P CMV
BGH pA
f1 ori
SV40 ori
Neomycin
SV40 pA
pUC ori
Ampicillin
pcDNA3.1(+)-Anti HBx P1#

Fig 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201110061840
- US 5449752 A
- US 5087616 A
- US 4450150 A
- EP 12757419 A
- CN 20111061840

### Non-patent literature cited in the description


- **SAMBROOK et al.** Molecular Cloning: A Laboratory Manual. Cold Spring Harbor Laboratory, 2001
- **HIGGINS ; SHARP.** *Gene,* 1988, vol. 73, 237-244
- **HEIN J.** *the Methods in Emzumology,* 1990, vol. 183, 625-645
- **DESHAYES et al.** *Cell. Mol. Life Sci.,* 2005, vol. 62, 1839-1849
- **EL-ANDALOUSSI et al.** *Curr. Pharm. Design.,* 2005, vol. 11, 3597-3611
- **CHAN et al.** Fmoc Solid Phase Peptide Synthesis. Oxford University Press, 2005
- **REID, R.** Peptide and Protein Drug Analysis. Marcel Dekker Company, 2000
- **SAMBROOK et al.** Molecular Cloning: A Laboratory Manual. Cold Spring Harbor Press, 2001
- **CAROLINE J.** Suicide Gene Therapy: Methods and Reviews. Humana Press, 2004
- **HUDECZ, F.** *Methods Mol Biol.,* 2005, vol. 298, 209-223
- **KIRIN et al.** *Inorg Chem.,* 2005, vol. 44 (15), 5405-5415
- **WADWA et al.** *J. Drug Targeting,* 1995, vol. 3, 111
- **WANG Q et al.** *Neoplasia.,* 2010, vol. 12 (2), 103-15
- **ZHANG WY et al.** *Acta Pharmacol Sinica.,* 2009, vol. 30 (8), 1153-61

SEQUENCE LISTING

<110> TIANJIN TOPTECH BIO-SCIENCE & TECHNOLOGY CO., LTD.

<120> POLYPEPTIDE DRUG AGAINST HEPATITIS B VIRUS X PROTEIN

<130> T31368EP

<140> EP12757419.2
<141> 2012-03-15

<150> CN20111061840
<151> 2011-03-15

<160> 16

<170> Patent In version 3.3

<210> 1
<211> 12
<212> PRT
<213> polypeptide

<400> 1

```
Gly Ser Ala Val Met Phe Ser Ser Lys Glu Arg Gly
1               5                   10
```

<210> 2
<211> 13
<212> PRT
<213> polypeptide

<400> 2

```
Ile Gly Ser Ala Val Met Phe Ser Ser Lys Glu Arg Gly
1               5                   10
```

<210> 3
<211> 13
<212> PRT
<213> polypeptide

<400> 3

```
Val Gly Ser Ala Val Met Phe Ser Ser Lys Glu Arg Gly
1               5                   10
```

<210> 4
<211> 12
<212> PRT
<213> polypeptide

<400> 4

```
Gly Ser Ala Val Met Phe Ser Ser Lys Asp Arg Gly
1               5                   10
```

<210> 5
<211> 12
<212> PRT
<213> polypeptide

<400> 5

```
Gly Ser Ala Val Met Phe Ser Ser Arg Glu Arg Gly
1               5                   10
```

<210> 6
<211> 12
<212> PRT
<213> polypeptide

<400> 6

```
Gly Ser Ala Val Met Phe Ser Ser Lys Glu His Gly
1               5                   10
```

<210> 7
<211> 36
<212> DNA
<213> Artificial Sequence

<400> 7
ggaagcgcag tgatgttttc cagtaaagaa cgcgga 36

<210> 8
<211> 39
<212> DNA
<213> Artificial Sequence

<400> 8
atcggaagcg cagtgatgtt ttccagtaaa gaacgcgga 39

<210> 9
<211> 39
<212> DNA
<213> Artificial Sequence

<400> 9
gtgggaagcg cagtgatgtt ttccagtaaa gaacgcgga 39

<210> 10

<211> 36
<212> DNA
<213> Artificial Sequence

<400> 10
ggaagcgcag tgatgttttc cagtaaagac cgcgga 36

<210> 11
<211> 36
<212> DNA
<213> Artificial Sequence

<400> 11
ggaagcgcag tgatgttttc cagtcgcgaa cgcgga 36

<210> 12
<211> 36
<212> DNA
<213> Artificial Sequence

<400> 12
ggaagcgcag tgatgttttc cagtaaagaa cacgga 36

<210> 13
<211> 57
<212> DNA
<213> forward DNA oligo

<400> 13
aattcatggg aagcgcagtg atgttttcca gtaaagaacg cggacgttgc acctgac 57

<210> 14
<211> 57
<212> DNA
<213> Reverse DNA oligo

<400> 14
tcgagtcagg tgcaacgtcc gcgttcttta ctggaaaaca tcactgcgct tcccatg 57

<210> 15
<211> 20
<212> DNA
<213> Forward Primer

<400> 15
taatacgact cactataggg 20

<210> 16
<211> 18
<212> DNA
<213> Reverse Primer

<400> 16

tagaaggcac agtcgagg 18

4